# EUROPEAN PATENT SPECIFICATION

(11) **EP 1 524 976 B1**
(45) Date of publication and mention of the grant of the patent: **13.09.2006**
(21) Application number: 03712575.4
(22) Date of filing: 13.03.2003
(51) Int. Cl.: A61K 31/40, A61K 31/445

(54) **A NON-HORMONAL APPROACH TO MALE CONTRACEPTION**
NICHT HORMONALE VERFARHEN ZUR MÄNNLICHER VERHÜTUNG
APPROCHE NON HORMONALE DE LA CONTRACEPTION MALE

(30) Priority: 13.03.2002 US 363561 P; 20.05.2002 US 381329 P
(43) Date of publication of application: 27.04.2005
(73) Proprietor: Unither Pharmaceuticals, Inc., Silver Spring, MD 20910 (US)
(72) Inventor: VAN DER SPOEL, Aarnoud, C., OX1 4RE (GB); JEYAKUMAR, Mylvaganam, Oxford, OX4 3HZ Oxfordshire (GB); BUTTERS, Terry, D., Oxfordshire OX44 4BS (GB); DWEK, Raymond, A., Oxford, Oxfordshire OX2 9AU (GB); PLATT, Frances, M., Oxfordshire OX8 8BN (GB)
(74) Representative: Warcoin, Jacques
(86) International application number: PCT/IB2003/001512
(87) International publication number: WO 2003/075916

(56) References cited:
- WO-A-02/055498
- VAN DER SPOEL ET AL: "Reversible infertility in male mice after oral administration of alkylated imino sugars. A nonhormonal approach to male contraception. " PNAS, vol. 99, no. 26, 2002, pages 17173-17178, XP002249591 USA

## Description

### CROSS REFERENCE TO RELATED APPLICATIONS

[0001A] This application claims priority based on U.S. Provisional Application No. 60/381,329 filed May 20,2002, and U.S. Provisional Application No. 60/363,561 filed March 13, 2002.

### FIELD OF THE INVENTION

**[0002B]** The present invention relates to a method of reversibly rendering a male mammal infertile by administration of a contraceptive specific to male mammals.

### BACKGROUND OF THE INVENTION

Glycosphingolipids (GSLs) are amphipathic molecules that reside in mammalian plasma membranes and are made up of the hydrophobic compound ceramide and one of many different oligosaccharide structures (see Svennerholm, (1994) *Prog Brain Res* **101**, XI-XIV for GSL nomenclature), GSLs have been implicated in multiple biological processes, including transmembrane signalling, cell adhesion, differentiation, migration and morphogenesis, oncogenic transformation and development of the nervous system (reviewed in Hakomori & Igarashi, (1993) *Adv Lipid Res* **25**, 147-62; Kopitz, Glycolipids: Structure and Function (1997) in Glycosciences, eds. Gabius, H. J. & Gabius, S. (Chapman & Hall, Weinheim), pp. 163-189; Kester, (1997) *Trends Glycosci Glycotech* **9**, 447-460; Lloyd & Furukawa (1998) *Glycoconj J 15,* 627-36; Hakomori, *et al,* (1998) *Glycobiology* **8**, xi-xix; Hakomori, (1998) *Acta Anat* (Basel) **161**, 79-90; Dickson, (1998) *Annu Rev Biochem* **67**, 27-48; Ledeen, *et al,* (1998) in Ann NY Acad Sci (NY Acad Sci, New York), Vol. 845; Schuette, et. al, (1999) *Biol Chem* **380**, 759-66). However, until recently, direct evidence for their biological significance, especially *in vivo,* has been hard to obtain due to a lack of cells and organisms that are deficient in GSLs. In the last few years, cDNA clones have been identified encoding glycosyltransferases that play key roles in GSL biosynthetic pathways, including ceramide glucosyltransferase, which catalyses the first step in the biosynthesis of all glucosylceramide-based GSLs (reviewed in Lloyd & Furukawa (1998) *Glycoconj J* **15,** 627-36; Ichikawa & Hirabayashi, (1998) *Trends Cell Biol* 8, 198-202). The establishment of a melanoma cell line deficient in the latter enzyme has shown that glucosylceramide-based GSLs are not essential for viability at the single-cell level (Ichikawa, *et al,* (1994) *Proc Natl Acad Sci U S A* 91, 2703-7). Murine embryonic stem cells lacking this enzyme can be induced to hematopoietic and neuronal differentiation *in vitro,* but do not form well-differentiated tissues *in vivo;* murine embyos lacking ceramide glucosyltransferase do not develop beyond the gastrula stage (Yamashita, *et al,* (1999) *Proc Natl Acad Sci U S A* **96,** 9142-7). Furthermore, mice, in which the gene for ganglioside GM2/GD2 synthase has been disrupted, and therefore devoid of all gangliosides more complex than GM3/GD3, develop normally, but exhibit axonal degeneration and reduced myelination in their nervous systems, and display deficits in motor behaviour later in life (Sheikh, *et al,* (1999) *Proc Natl Acad Sci USA* **96**, 7532-7). Unexpectedly, the absence of complex gangliosides in these mice is also associated with impaired spermatogenesis, and leads to male infertility (Takamiya, *et al,* (1998) *Proc Natl Acad Sci U S A* **95,** 12147-52).

An alternative, non-genetic approach to probe GSL functions is to manipulate their levels with titratable inhibitors of GSL biosynthesis (reviewed/described in (Radin, *et al,* (1993) *Adv Lipid Res* **26,** 183-213; Platt & Butters, (1995) *Trends Glycosci Glycotech* 7,495-511; Kolter & Sandhoff, (1996) *Chem Soc Rev* **25,** 371-381; Inokuchi, (1997) *Trends Glycosci Glycotech* **9** Supplement, S37-S45; van Echten-Deckert, *et al,* (1998) *J Biol Chem* **273,** 1184-91; Lee, *et al,* (1999) *J Biol Chem* **274***,* 14662-9); compounds with this characteristic may have several clinical applications (reviewed in Platt & Butters, (1998) *Biochem Pharmacol* **56***,* **421-30;** Radin, (1999) *Biochem Pharmacol* **57**, 589-95). It has previously been established that alkylated iminosugars reduce the rate of synthesis of various glucosylceramide-based GSLs by inhibiting ceramide glucosyltransferase (Platt, *et al,* (1994) *J Biol Chem* **269,** 8362-5; Platt, *et al,* (1994) *J Biol Chem* **269,** 27108-14). These compounds have the unique advantage that they can be orally administered to experimental animals over a wide dose range and are well tolerated even at high dosage levels, allowing extensive and prolonged attenuation of GSL biosynthesis (Platt, *et al,* (1997) *J Biol Chem* **272,** 19365-72; Andersson, *et al,* (2000) *Biochem Pharmacol* **59**: 821-29: Dwek, R. A., Butters, T. D., Platt, F. M. & Zitzmann, N. (2002) *Nature Reviews Drug Discovery* **1**, 65-75). The alkylated iminosugar N-butyldeoxynojirimycin (NB-DNJ) is a potent inhibitor of alpha-glucosidase 1 (involved in N-glycan synthesis), and an even more potent inhibitor of glucosylceramide glucosyltransferase and is well tolerated in humans (Fischl, *et al* (1994) *J Acquir Immune Defic Syndr* 7, 139-47). The inventors have previously found that NB-DNJ is therapeutically effective in mouse models with impaired GSL degradation (Platt, *et al,* (1997) *Science* 276, 428-31; Jeyakurnar, *et al,* (1999) *Proc Natl Acad Sci U S A* 96, 6388-93).

The susceptibility of spermatogenesis to hormonal control has attracted much attention in the development of a male contraceptive (Amory, *et al,* (2000) *Trends Endocrinol Metab* 11, 61-66; Anderson, (2000) *Br Med Bull* 56, 717-728; Handelsman, (2000) *Int J Androl* 23 Suppl 2, 8-12; Oxynos, *et a*/*,* (2000) *Baillieres Best Pract Res Clin Endocrinol Metab* 14, 473-487). Men can become azoospermic, and thus infertile, through suppression of gonadotropin (LH and FSH) secretion from the pituitary. This can be achieved by administration of progestogens, anti-androgens, 5alpha-reductase inhibitors or gonadotrophin-releasing hormone antagonists. Current problems with these approaches are the following (Anderson, (2000) *supra*): First, they are not completely effective; complete suppression of spermatogenesis is not always achieved. Second, the lack of the pituitary gonadotropins results in a decrease of the testicular testosterone production, necessitating testosterone replacement. The currently available testosterone preparations are not entirely acceptable. Third, the exogenous testosterone has a number of undesirable metabolic effects. Finally, most compounds evaluated as a male contraceptive as well as most testosterone preparations need to be injected intramuscularly, or implanted subdermally. Thus, there remains a continued need in the art for non-endocrinal approaches for pharmacological intervention in the generation and maturation of male germ cells. The present invention satisfies this objective by providing a non-hormonal method of male contraception.

The patent application WO 02/055498 which is a prior art according to article 54(3) EPC describes the compounds of formula 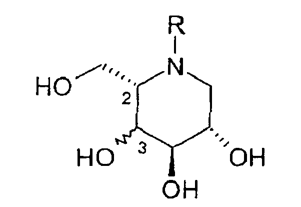 wherein R is C₁₋₁₆ straight or branched-chain alkyl, optionally substituted by C₃₋₇ cycloalkyl, and optionally interrupted by -O- the oxygen being separated from the ring nitrogen by at least two carbon atoms, or C₁₋₁₀ alkylaryl where aryl is phenyl, pyridyl, thienyl or furyl wherein phenyl is optionally substituted by one or more substituents selected from F, Cl, Br, CF₃, OCF₃, OR¹, and C₁₋₆ straight or branched-chain alkyl; and
R¹ is hydrogen, or C₁₋₆ straight or branched-chain alkyl;
and their use in reversibly rendering a male mammal infertil.

### SUMMARY OF THE INVENTION

The present invention provides a method for reversibly rendering a male mammal infertile by administering to such a mammal a pharmaceutical composition comprising an N-substituted imino sugar compound. The imino sugar compounds may be represented by Formula I whith the proviso of claim 1 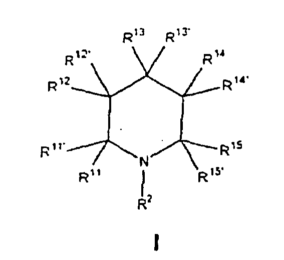 wherein each of R¹¹, R^{11'}, R¹², R^{12'}, R¹³, R^{13'}, R¹⁴, R^{14'}, R¹⁵ and R^{15'}, is selected, independently from each other, from the group consisting of -H; -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy; aryloxy; aralkoxy; -(alkylene)oxy(alkyl); -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; -NHOH; aryl, and heteroaryl in which at least two of R¹¹, R^{11'}, R¹², R^{12'}, R¹³, R^{13'}, R¹⁴, R^{14'}, R¹⁵ and R^{15'} is selected for a -CH₃,-CH₂OH and -OH

R² is a substituent selected from the group consisting of linear or branched C₁₋₁₈ alkyl, C₂₋₁₈ alkenyl and alkynyl; and aralkyl.

Each alkyl, alkenyl, alkynyl, and aryl moiety may be optionally substituted with one or more groups independently selected from the group consisting of -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy, aryloxy; aralkoxy; -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; and -NHOH with the proviso that the compounds of formula I or their pharmaceutically acceptable salt thereof do not have the following formula 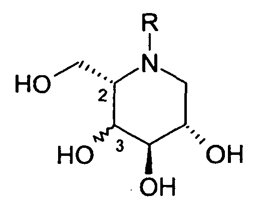 wherein R is C₁₋₁₆ straight or branched-chain alkyl, optionally substituted by C₃₋₇ cycloalkyl, and optionally interrupted by -O- the oxygen being separated from the ring nitrogen by at least two carbon atoms, or C₁₋₁₀ alkylaryl where aryl is phenyl, pyridyl, thienyl or furyl wherein phenyl is optionally substituted by one or more substituents selected from F, Cl, Br, CF₃, OCF₃, OR¹, and C₁₋₆ straight or branched-chain alkyl; and
R¹ is hydrogen, or C₁₋₆ straight or branched-chain alkyl;

According to one aspect of this invention, the composition is administered for a time sufficient to render the male mammal infertile. After treatment, when the composition is no longer administered, the male mammal regains fertility.

According to another aspect of this invention, the composition is administered to render a male mammal infertile, withdrawn from the mammal whereby it fully regains fertility, then administered once again to render the mammal infertile.

Administration of the imino sugar compounds of this invention causes male mice to be sterile, but has no effect on female fertility.
Thus, the imino sugar compounds of this invention effectively serve as male contraceptives.

### BRIEF DESCRIPTION OF THE DRAWINGS

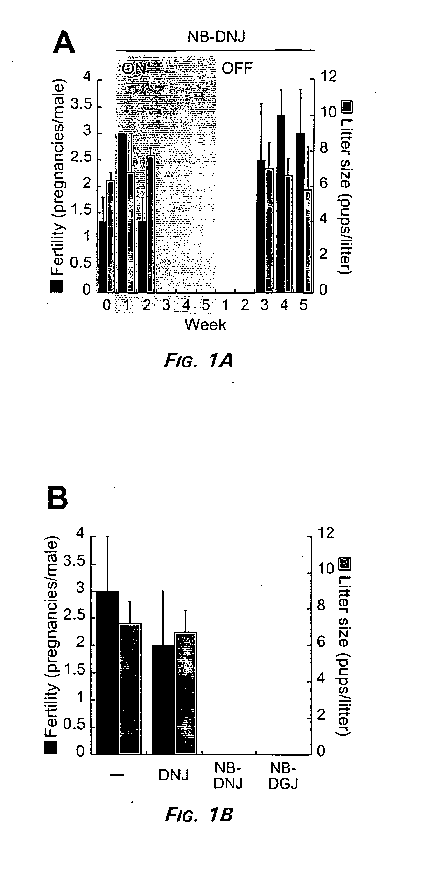

FIGURE 1A shows the effects of oral administration and withdrawal of *N*B-DNJ on fertility of male mice. FIGURE 1A shows that animals were either not treated or else given *N*B-DNJ at 2400 mg/kg per day for one to five weeks, after which *N*B-DNJ was no longer administered. FIGURE 1B shows the effects of *N*B-DNJ and *N*B-DGJ on pregnancies per male after each of the three males was caged with four female mice for one week.

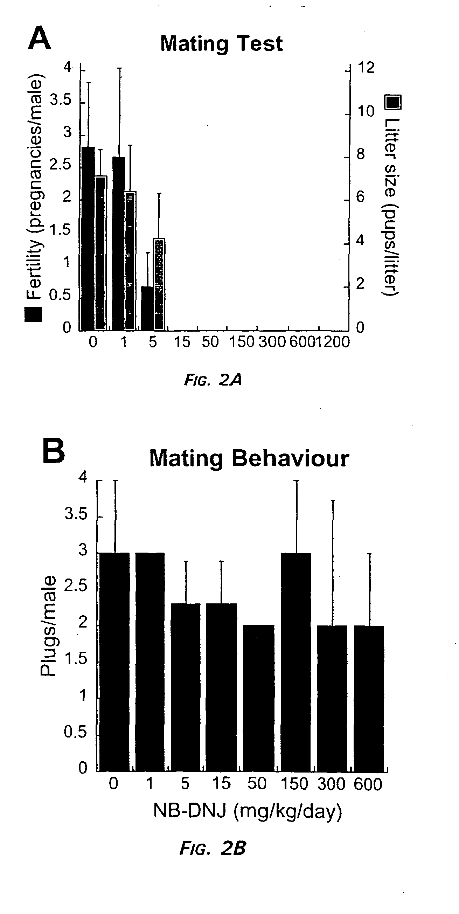

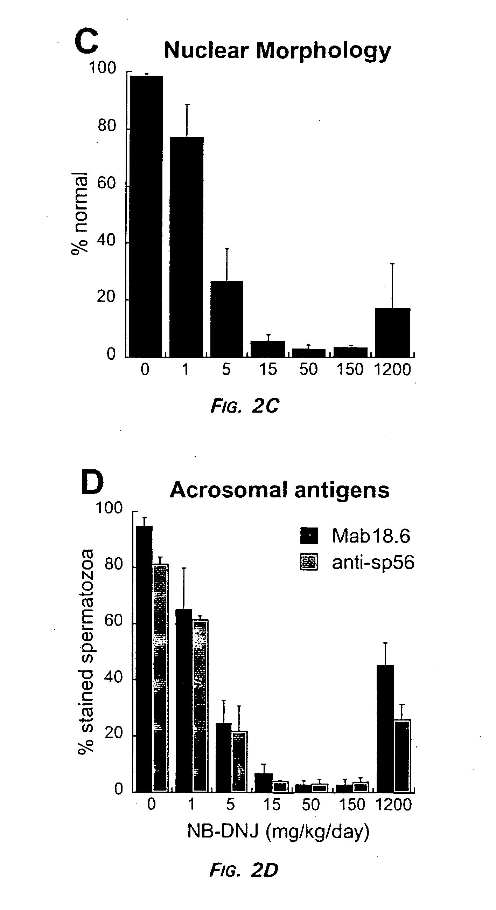

FIGURES 2A, 2B, 2C, AND 2D show the dose dependence of induction of infertility in male mice through oral administration of *N*B-DNJ. FIGURE 2A compares the pregancies per male with the dose of *N*B-DNJ after the males were fed diets containing *N*B-DNJ for five weeks and then caged with four female mice each for nine days. FIGURE 2B compares the number of vaginal plugs per male with the dose (not determined for 1200 mg/kg day). FIGURE 2C compares the percentage of abnormal nuclear morphology with dose observed after epididymal spermatozoa were examined. FIGURE 2D compares the number of cells immunofluorescing due to acrosomal antigens with dose (not determined, for 300 and 600 mg/kg day).

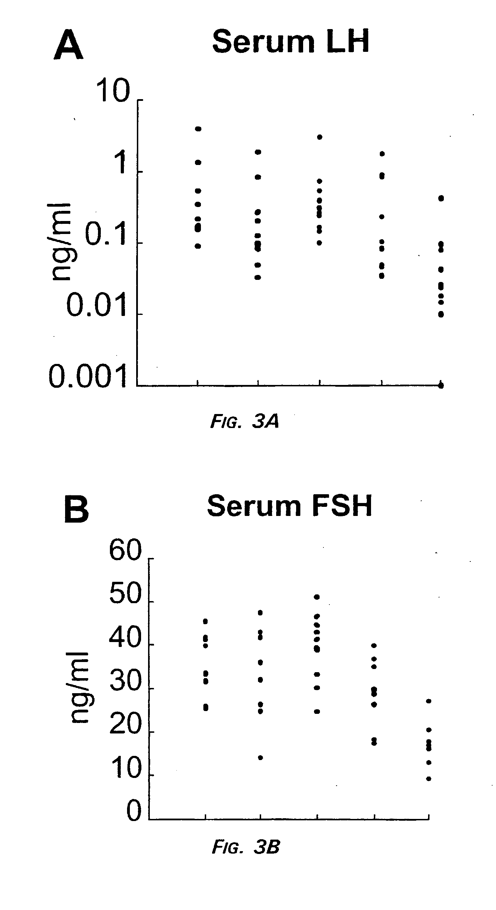

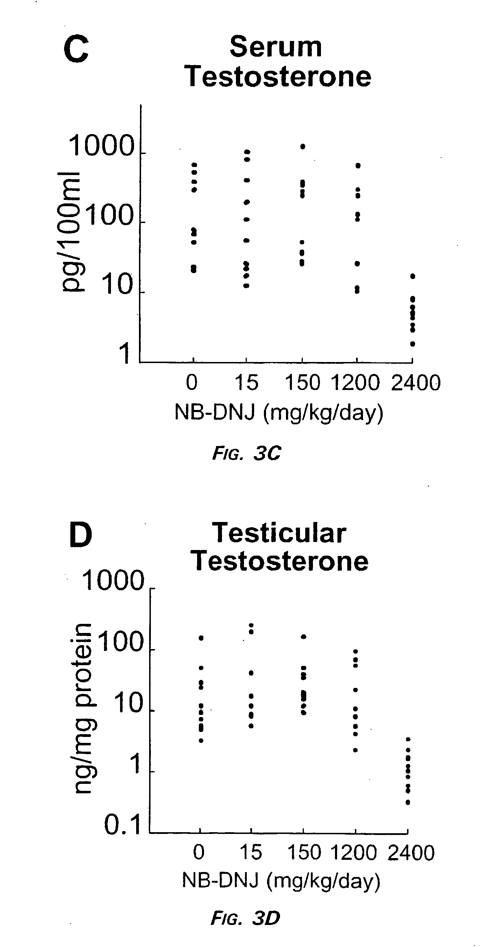

FIGURES 3A, 3B, 3C, AND 3D shows the effect of *N*B-DNJ at four different doses on the reproductive endocrinology of individual male mice. FIGURE 3A shows the effect on luteinizing hormone; FIGURE 3B shows the effect on follicle stimulating hormone; FIGURE 3C shows the effect on serum testosterone; and FIGURE 3D shows the effect on testicular testosterone.

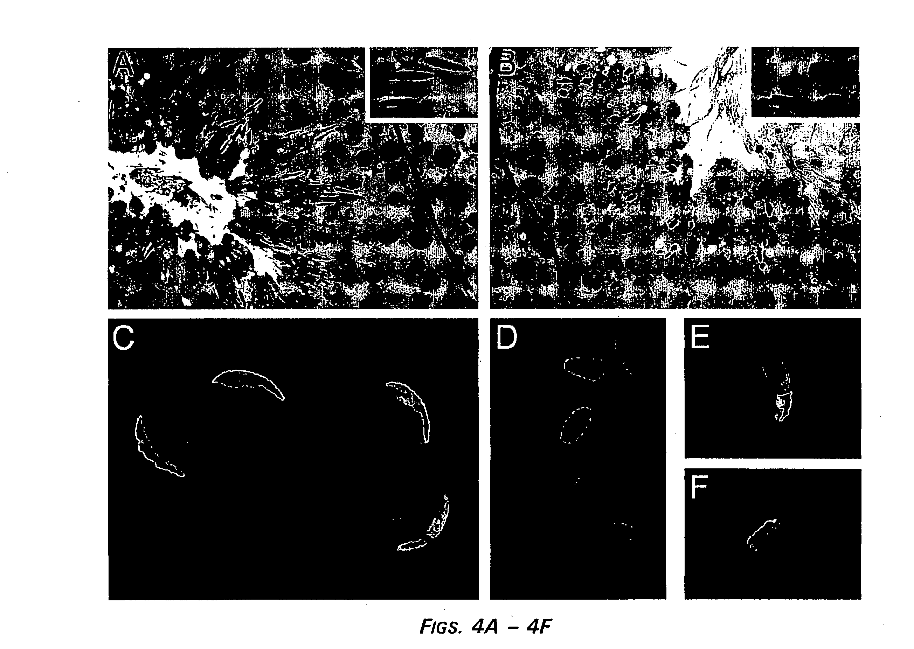

FIGURES 4A, 4B, 4C, 4D, 4E, and 4F compare the morphological features of spermatozoa from normal mice with those from mice treated with *N*B-DNJ. FIGURE 4A shows sections from stage I seminiferous tubules from a control mouse; FIGURE 4B shows the sections in a mouse treated with 50 mg/kg/day *N*B-DNJ; and the insets show close-ups of step 13 spermatid heads. Figures 4C, 4D, 4E and 4F show epididymal spermatozoa that were incubated with anti-acrosomal monoclonal 18.6 and counterstained with the nuclear dye Hoechst 33342. Figure 4C shows epididymal spermatozoa from normal mice, while Figures 4D, 4E, and 4F show epididymal spermatozoa from mice treated with 150 mg/kg/day. The original magnifications were 1000x.

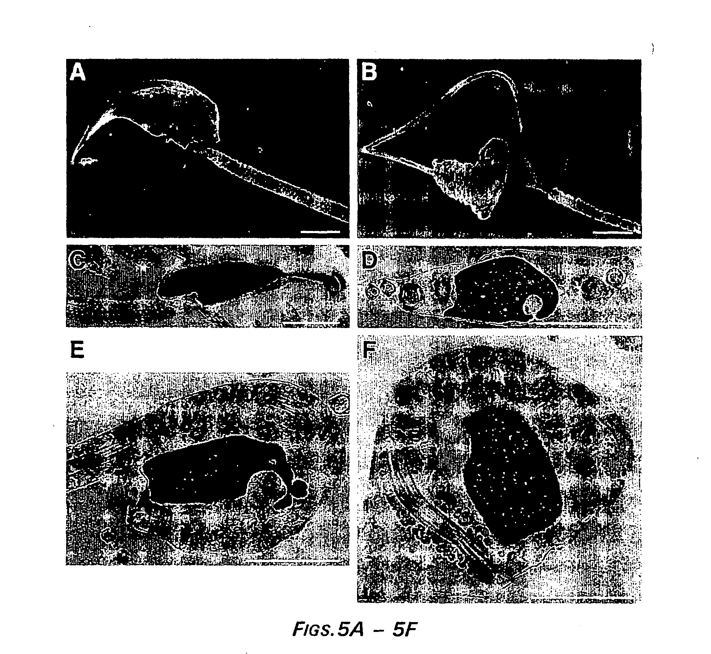

FIGURES 5A and 5C show the morphological features of epididymal spermatozoa from normal mice, while FIGURES 5B, 5D, 5E, AND 5F show the same in *N*B-DNJ-treated mice (1,200 mg/kg/day). FIGURE 5A shows a normal head and midpiece and FIGURE 5B shows a spatulate sperm head with a disorganized squat mitochondrial sheath. FIGURES 5C shows a normal spermatozoon and FIGURES D, E and F show spermatozoa with alterations in nuclear shape, tail orientation, and morphology and arrangement of mitochondria. The bar represents 2 µm.

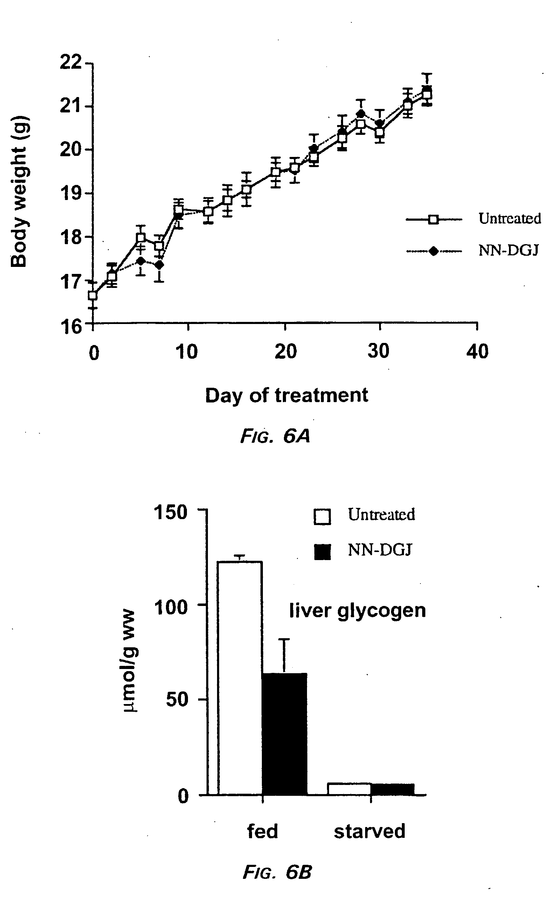

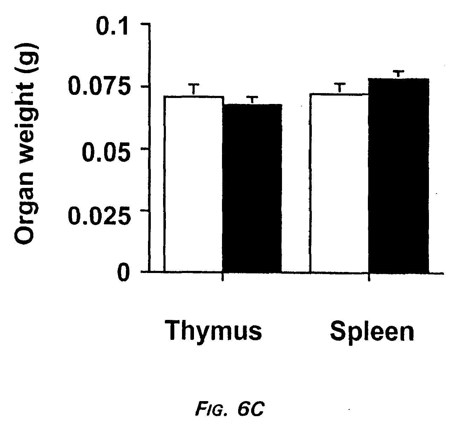

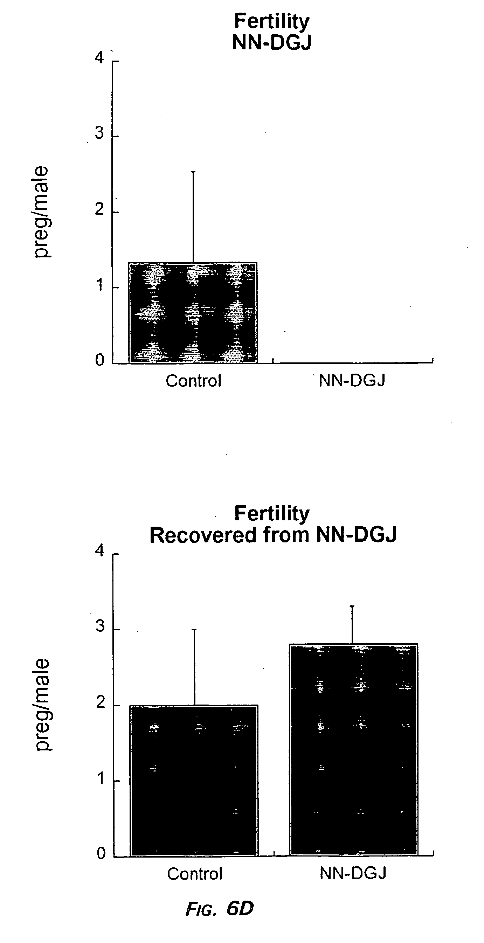

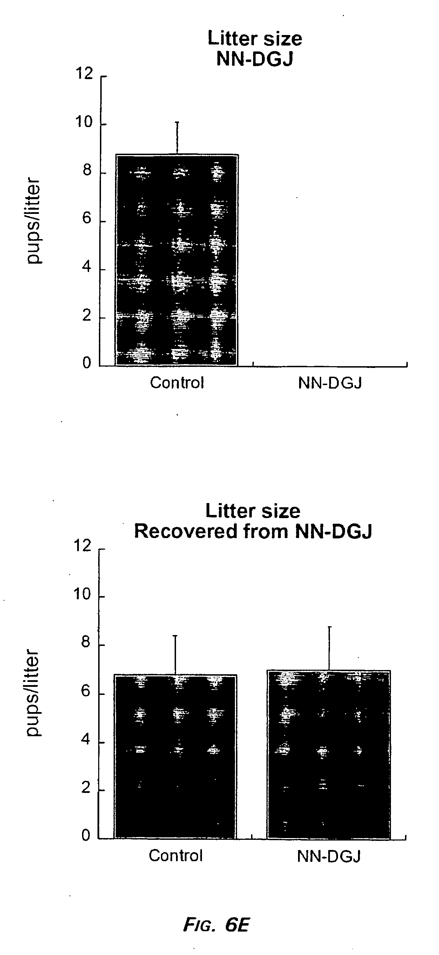

FIGURES 6A, 6B, 6C, 6D, AND 6E show the effects of *N*N-DGJ (250 mg/kg/day) on male mice. FIGURE 6A shows that the weight gain of eight *N*N-DGJ-treated mice was the same as for untreated mice. FIGURE 6B compares liver lycogen levels in *N*N-DGJ-treated and untreated mice. FIGURE 6C compares the thymus and spleen weights between *N*N-DGJ-treated and untreated mice. FIGURE 6D shows the fertility of four male mice after five weeks of treatment with *N*N-DGJ, and again at six weeks after treatment was stopped. Similarly, FIGURE 6E demonstrates the effect of *N*N-DGJ on the litter size after five weeks of treatment and again after six weeks had elapsed since treatment was stopped.

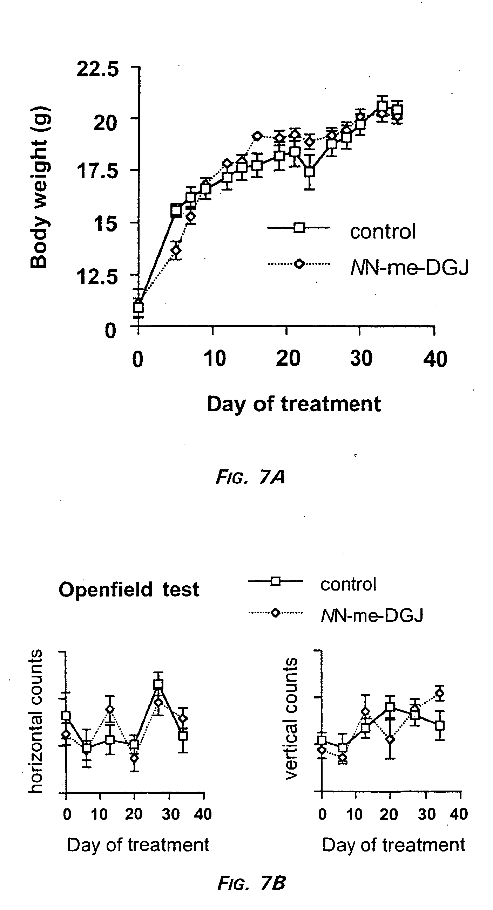

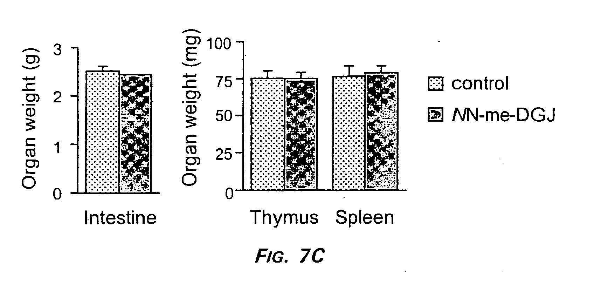

FIGURES 7A, 7B, AND 7C shows the effects of *N*N-6-MeDGJ (250 mg/kg/day) on male mice. FIGURE 7A compares the weight gain of *N*N-6-MeDGJ-treated mice to that for untreated mice. FIGURE 7B shows that the spontaneous horizontal and vertical movements of *N*N-6-MeDGJ-treated mice in an openfield test did not differ from those of untreated mice. FIGURE 7C compares the intestinal tract, thymus, and spleen weights between *N*N-6-MeDGJ-treated and untreated mice.

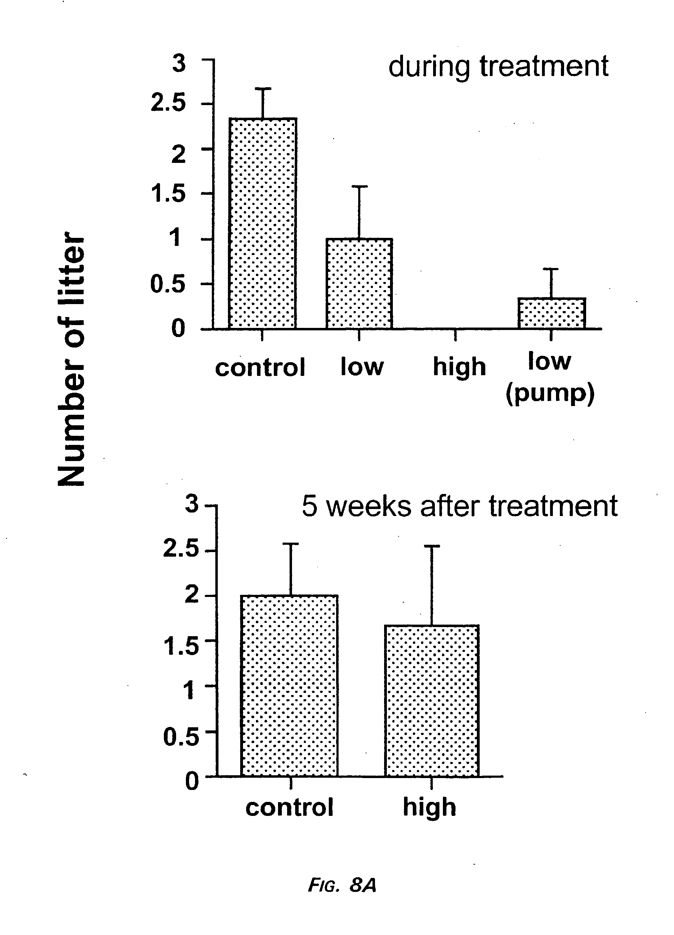

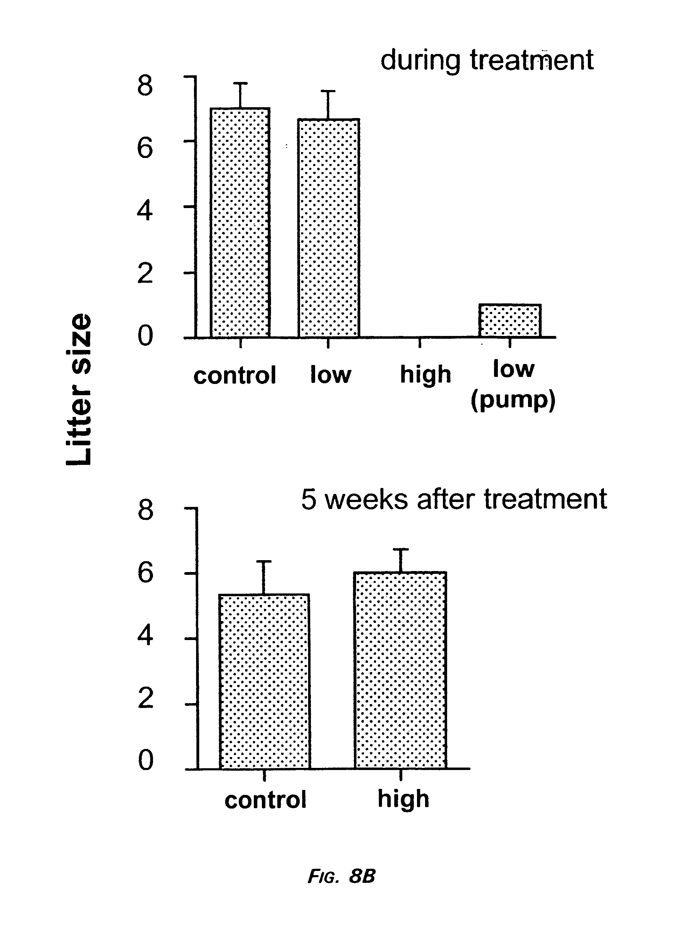

FIGURES 8A AND 8B demonstrate the effect of *N*N-6-MeDGJ on the fertility of male mice (15 mg/kg/day, low, low(pump); or 250 mg/kg/day, high). FIGURE 8A compares the number of litters, and FIGURE 8B compares the litter size, at each dosage regimen during treatment to that at 5 weeks after treatment.

### DEFINITIONS

Unless otherwise specified, the terms "a" or "an" mean "one or more."

Unless otherwise specified, the term "alkyl" as used herein refers to straight- and branched-chain alkyl radicals containing 1, 2, 3, 4, 5, or 6 carbon atoms and includes, for example, methyl, ethyl, butyl, and nonyl.

The term "aryl" as used herein refers to a monocyclic aromatic group such as phenyl or a benzo-fused aromatic group such as indanyl, naphthyl, or fluorenyl and the like.

The term "heteroaryl" refers to aromatic compounds containing one or more hetero atoms. Examples include as pyridyl, furyl, and thienyl or a benzofused aromatic containing one or more heteroatoms such as indolyl or quinolinyl.

The term "heteroatom" as used herein refers to non-carbon atoms such as N, O, and S.

The term "cycloalkyl" as used herein refers to a carbocyclic ring containing 3, 4, 5, 6, 7, or 8 carbons and includes, for example, cyclopropyl and cyclohexyl.

Unless otherwise specified, the term "alkoxy" as used herein refers to a straight- or branched-chain alkoxy containing 1, 2, 3, 4, 5 or 6 carbon atoms and includes, for example, methoxy and ethoxy.

The term "alkenyl" as used herein refers to a straight or branched-chain alkyl containing one or more double bonds such as propenyl and vinyl.

The term "aralkyl" as used herein refers to an alkyl substituted with an aryl such as benzyl and phenethyl.

The term "alkynyl" as used herein refers to a straight or branched-chain alkyl containing one or more triple bonds such as ethynyl and propynyl.

The term "aryloxy" as used herein refers to a substituent created by replacing the hydrogen atom in an -OH group with an aryl group, and includes, for example, phenoxy.

The term "aralkoxy" as used herein refers to an alkoxy group substituted with an aryl group, such as 2-phenylethoxy.

The term "alkylamino" as used herein refers to an amino group substituted with one alkyl group such as methylamino (-NHCH₃) and ethylamino (-NHCH₂CH₃).

The term "dialkylamino" as used herein refers to an amino group substituted with two alkyl groups such as dimethylamino (-N(CH₃)₂) and diethylamino (-N(CH₂CH₃)₂).

### DETAILED DESCRIPTION OF THE PREFERRED EMBODIMENTS

The compounds employed in the method of this invention are effective as male contraceptives. In the broadest aspect, the compounds are represented by Formula I with the proviso of claim 1.

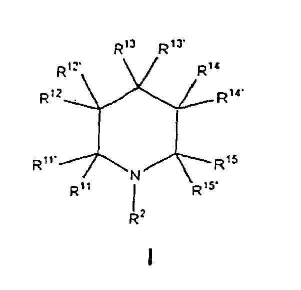

Each of R¹¹, R^{11'}, R¹², R^{12'}, R¹³, R^{13'}, R¹⁴, R^{14'}, R¹⁵, and R^{15'} is selected, independently from the other, from the group consisting of -H; -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy; aryloxy; aralkoxy; -(alkylene)oxy(alkyl); -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; -NHOH; aryl, and heteroaryl in which at least two of R¹¹, R^{11'}, R¹², R^{12'}, R¹³, R^{13'}, R¹⁴, R^{14'}, R¹⁵, and R^{15'} being selected from -CH₃, -CH₂OH, and -OH.

In a preferred embodiment, at least one of R¹¹, R^{11'}, R¹², R^{12'}, R¹³, R^{13'}, R¹⁴, R^{14'}, R¹⁵, and R^{15'} is a hydroxymethyl group (-CH₂OH). Alternatively, at least one of R¹¹, R^{11'}, R¹², R^{12'}, R¹³, R^{13'}, R¹⁴, R^{14'}, R¹⁵, and R^{15'} is a hydroxy group (-OH).

R² is a substituent selected from linear or branched C₁₋₁₈ alkyl, C₂₋₁₈ alkenyl and alkynyl, and aralkyl, optionally substituted with one or more groups independently selected from -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy, aryloxy; aralkoxy; -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; and -NHOH.

Preferably, R² is an optionally substituted linear or branched C₁₋₁₈ alkyl group. Even more preferably, R² is an optionally substituted linear or branched C₄₋₉ alkyl group. Examples of R² include alkyl substituents such as methyl, ethyl, propyl, butyl, pentyl, hexyl, heptyl, octyl, and nonyl. An example of R² being a substituted alkyl group is 7-oxanonanyl (-CH₂(CH₂)₅₋O-CH₂CH₃). Most preferably, R² is *n*-butyl, *n*-nonyl, or 7-oxanonyl.

In certain embodiments of Formula I, the compound may be represented as one of the following formulae: 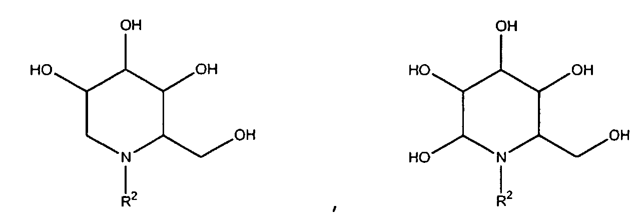 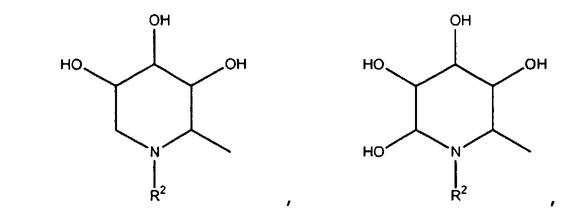 and

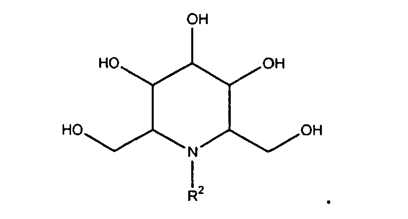

The stereochemistry at each ring carbon atom in the formulae above may vary independently from that in the other ring carbon atoms. More preferably, the compound has one of the formulae as set forth in Table I:

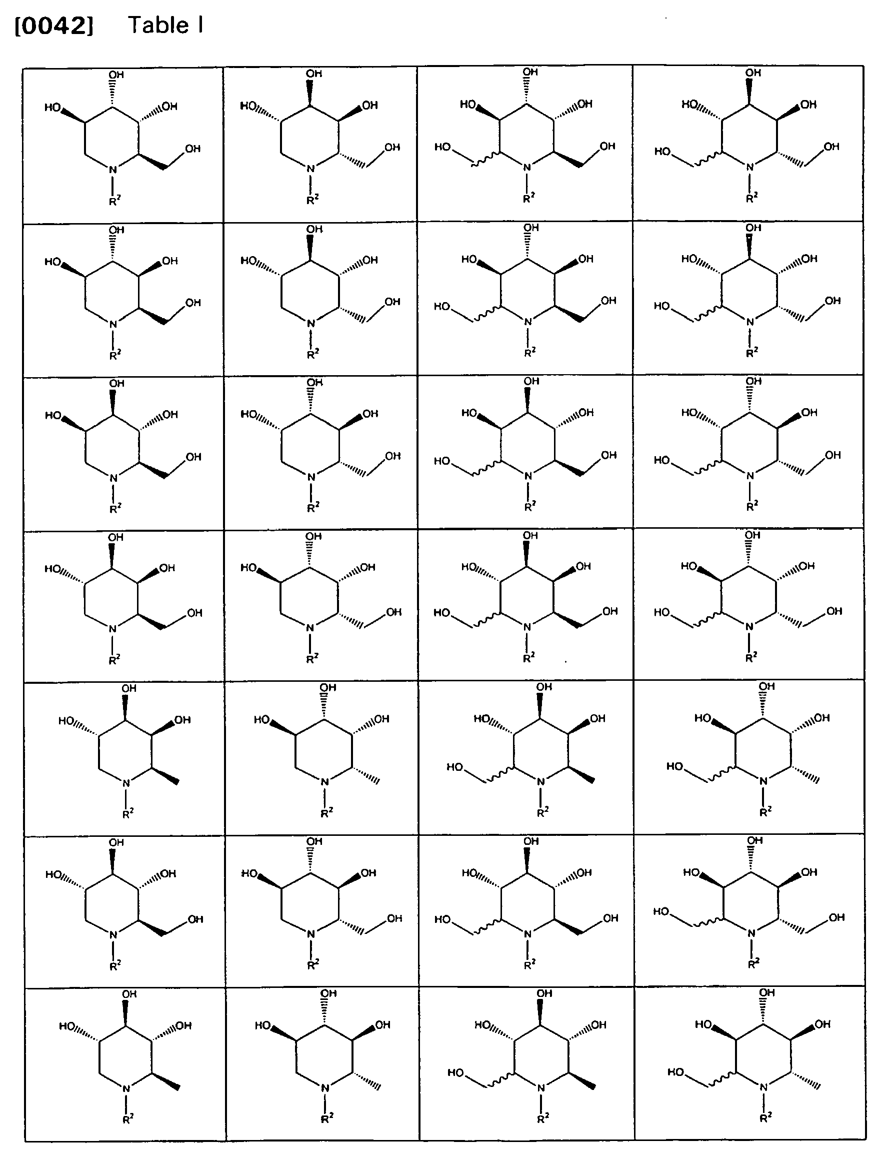

Still more preferably, the compound is *N*-alkyl substituted wherein R² is a linear or branched C₁₋₁₈ alkyl group optionally substituted with a C₁₋₆ alkoxy group. The most preferred compounds include *N-*butyldeoxygalactonojirimycin (*N*B-DGJ), *N*-butyldeoxynojirimycin (*N*B-DNJ), *N*-nonyldeoxynojirimycin (*N*N-DNJ), *N*-nonyl-6-methyldeoxynojirimycin(*N*N-6-MeDNJ), and *N*-7-oxanonyl-6-methyldeoxygalactonojirimycin (*N*-7*-*oxanonyl-6-MeDGJ):

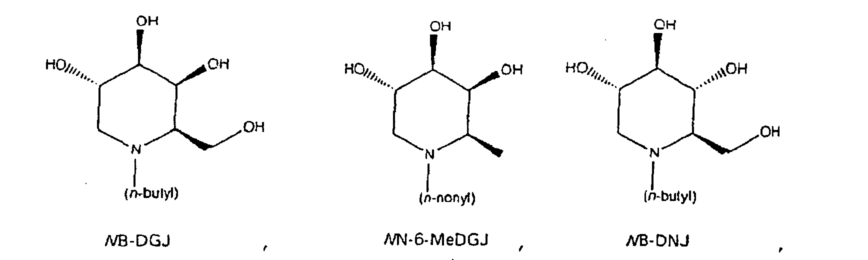

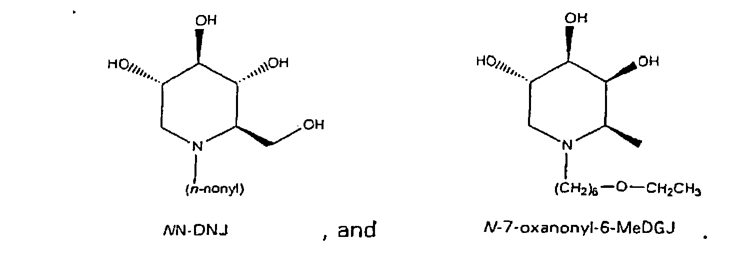

In yet another embodiment, the present invention provides a method wherein the first step a therapeutically effective amount of a compound described above is administered, as a pharmaceutical composition, to a fertile male mammal. The length of administration of the composition is sufficient to render the male mammal infertile so long as the composition is administered. Upon withdrawal of the composition in a second step, the male mammal undergoes a complete reversal to its previous state of fertility. In a preferred embodiment, the first and second steps can be repeated sequentially, thereby subjecting the male mammal to alternating periods of infertility and fertility for as long as desired.

Still a further embodiment of this invention provides a method as described immediately above, but with the additional third step of resuming administration of a composition of this invention for a time sufficient to once again render a male mammal infertile. In a preferred embodiment, the three steps can be repeated sequentially. In this manner, the male mammal undergoes periods of infertility, followed by fertility, and finally infertility.

The present invention is applicable to all male mammals. In particular, it is applicable to humans, and to companion mammals including dogs, cats, and horses. In addition, it is applicable to economically important mammals such as ungulates, particularly cattle, sheep, goats, water buffalo, camels and pigs, as well as, for example, apes, monkeys, llamas, rodents (e.g. rats or mice) and rabbits.

### Preparation of the Compounds

The compounds of this invention may be prepared from commercially available imino compounds used as starting materials. Commercial sources include Sigma, St. Louis, MO; Cambridge Research Biochemicals, Norwich, Cheshire, United Kingdom; and Toronto Research Chemicals, Ontario, Canada.

Alternatively, the present compounds can be prepared by synthetic methods known to those skilled in the art. For example, the syntheses of a variety of deoxynojirimycin (DNJ) derivatives are described in U.S. Patent Nos. 5,622,972; 5,200523; 5,043,273; 4,944,572; 4,246,345; 4,266,025; 4,405,714; and 4,806,650. Exemplary methods and processes for the production of N-butyldeoxynojirimycin (NB-DNJ) are set forth in EP-B-0012278; EP-A-0624652; U.S. Pat. Nos. 4,182,767; 4,266,025; 4,405,714; and 5,151,519. N-butyldeoxygalactonojirimycin (*N*B-DGJ) can be produced as described in Platt *et al.,* (1994), *J. Biol. Chem.,* **269**:27108-14. Other imino sugar compounds described herein are known and may be prepared by methods set forth in U.S. Pat. Nos. 4,861,892; 4,894,388; 4,910,310; 4,996,329; 5,011,929; 5,013,842; 5,017,704; 5,580,884; 5,286,877; 5,100,797; and 6,291,657.

### Pharmaceutical Compositions

The compounds of the invention can be formulated for a variety of modes of administration. Techniques and formulations generally may be found in REMINGTON'S PHARMACEUTICAL SCIENCES (1 8th ed.), Mack Publishing Co. (1990).

The medicaments may be adapted for administration by any appropriate route, for example by the oral (including buccal or sublingual), rectal, nasal, topical (including buccal, sublingual or transdermal), or parenteral (including subcutaneous, intramuscular, intravenous or intradermal) route although oral administration is preferred. Such a composition may be prepared by any method known in the art of pharmacy, for example by admixing the active ingredient with a carrier under sterile conditions.

Pharmaceutically acceptable salts are generally well known to those of ordinary skill in the art, and may include, by way of example but not limitation, acetate, benzenesulfonate, besylate, benzoate, bicarbonate, bitartrate, bromide, calcium edetate, camsylate, carbonate, citrate, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, mucate, napsylate, nitrate, pamoate (embonate), pantothenate, phosphate/disphosphate, polygalacturonate, salicylate, stearate, subacetate, succinate, sulfate, tannate, tartrate, or teoclate. Other pharmaceutically acceptable salts may be found, for example, in REMINGTON'S PHARMACEUTICAL SCIENCES (18th ed.), *supra.*

Preferred pharmaceutically acceptable salts include, for example, acetate, benzoate, bromide, carbonate, citrate, gluconate, hydrobromide, hydrochloride, maleate, mesylate, napsylate, pamoate (embonate), phosphate, salicylate, succinate, sulfate, or tartrate.

For injection, the agents of the invention may be formulated in aqueous solutions, preferably in physiologically compatible buffers such as Hank's solution, Ringer's solution, or physiological saline buffer. For such transmucosal administration, penetrants appropriate to the barrier to be permeated are used in the formulation. Such penetrants are generally known in the art.

Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the invention to be formulated as tablets, pills, capsules, liquids, gels, syrups, slurries, suspensions and the like, for oral ingestion by a patient to be treated.

Pharmaceutical compositions suitable for use in the present invention include compositions wherein the active ingredients are contained in an effective amount to achieve its intended purpose. Determination of the effective amounts is well within the capability of those skilled in the art, especially in light of the detailed disclosure provided herein.

In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions.

The medicaments or contraceptives described herein and which are also for use in the methods provided herein, may include one or more of the following: preserving agents, solubilising agents, stabilising agents, wetting agents, emulsifiers, sweeteners, colorants, odourants, salts, buffers, coating agents or antioxidants. They may also contain therapeutically active agents in addition to the compounds and/or agents described herein. Pharmaceutical preparations for oral use can be obtained by combining the active compounds with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations, for example, maize starch, wheat starch, rice starch, potato starch, gelatin, gum tragacanth, methyl cellulose, hydroxypropylmethyl-cellulose, sodium carboxymethyl-cellulose (CMC), and/or polyvinylpyrrolidone (PVP: povidone). If desired, disintegrating agents may be added, such as the cross-linked polyvinylpyrrolidone, agar, or alginic acid or a salt thereof such as sodium alginate.

Pharmaceutical preparations which can be used orally include push-fit capsules made of gelatin, as well as soft, sealed capsules made of gelatin, and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols (PEGs). In addition, stabilizers may be added.

### Routes of Administration

Various routes of administration will now be considered in greater detail:

### Oral Administration

Medicaments adapted for oral administration may be provided as capsules or tablets; as powders or granules; as solutions, syrups or suspensions (in aqueous or non-aqueous liquids); as edible foams or whips; or as emulsions.

Tablets or hard gelatine capsules may comprise lactose, maize starch or derivatives thereof, stearic acid or salts thereof.

Soft gelatine capsules may comprise vegetable oils, waxes, fats, semi-solid, or liquid polyols etc.

Solutions and syrups may comprise water, polyols and sugars. For the preparation of suspensions oils (e.g. vegetable oils) may be used to provide oil-in-water or water-in-oil suspensions.

### Transdermal Administration

Medicaments adapted for transdermal administration may be provided as discrete patches intended to remain in intimate contact with the epidermis of the recipient for a prolonged period of time. For example, the active ingredient may be delivered from the patch by iontophoresis (Iontophoresis is described in *Pharmaceutical Research,* 3(**6**):318 (1986)).

### Topical Administration

Medicaments adapted for topical administration may be provided as ointments, creams, suspensions lotions, powders, solutions, pastes, gels, sprays, aerosols or oils.

For infections of the eye or other external tissues, for example mouth and skin, a topical ointment or cream is preferably used. When formulated in an ointment, the active ingredient may be employed with either a paraffinic or a water-miscible ointment base. Alternatively, the active ingredient may be formulated in a cream with an oil-in-water base or a water-in-oil base.

Medicaments adapted for topical administration to the eye include eye drops. Here the active ingredient can be dissolved or suspended in a suitable carrier, e.g. in an aqueous solvent.

Medicaments adapted for topical administration in the mouth include lozenges, pastilles and mouthwashes.

### Rectal Administration

Medicaments adapted for rectal administration may be provided as suppositories or enemas.

### Nasal Administration

Medicaments adapted for nasal administration which use solid carriers include a coarse powder (e.g. having a particle size in the range of 20 to 500 microns). This can be administered in the manner in which snuff is taken, i.e. by rapid inhalation through the nose from a container of powder held close to the nose.

Compositions adopted for nasal administration which use liquid carriers include nasal sprays or nasal drops. These may comprise aqueous or oil solutions of the active ingredient.

Medicaments adapted for administration by inhalation include fine particle dusts or mists, which may be generated by means of various types of apparatus, e.g. pressurised aerosols, nebulisers or insufflators. Such apparatus can be constructed so as to provide predetermined dosages of the active ingredient.

### Parenteral Administration

Medicaments adapted for parenteral administration include aqueous and non-aqueous sterile injectable solutions or suspensions. These may contain antioxidants, buffers, bacteriostats and solutes which render the compositions substantially isotonic with the blood of an intended recipient. Other components which may be present in such compositions include water, alcohols, polyols, glycerine and vegetable oils, for example. Compositions adapted for parenteral administration may be presented in unit-dose or multi-dose containers, for example sealed ampoules and vials, and may be stored in a freeze-dried (lyophilised) condition requiring only the addition of a sterile liquid carrier, e.g. sterile water for injections, immediately prior to use. Extemporaneous injection solutions and suspensions may be prepared from sterile powders, granules and tablets.

### Dosages

Dosages will be readily determinable by routine trials, and will be under the control of the physician or clinician. The guiding principle for determining a suitable dose will be delivery of a suitably efficacious but non-toxic, or acceptably toxic, amount of material. For *N*B-DNJ or a similar compound, a daily dosage for an adult could be expected to be in the range of from 0.1 mg to 5 g of active agent, and may be in the range of from 3 to 2400 mg, preferably 5 to 800 mg, more preferably 15 to 400 mg, and most preferably 15 to 100 mg. The dosage may be administered in a single daily dose or alternatively in two, three or more doses during the day.

The following examples are proffered merely to illustrate the invention described above; they are not intended to limit in any way the scope of this invention. Throughout the specification, any and all references to publicly available documents are specifically incorporated into this patent application by reference.

### EXAMPLES

The following examples show that male, but not female, mice became sterile following at least three weeks of oral administration of the compounds of the present invention, and that fertility was regained in the fourth week after drug withdrawal. Testicular weights and serum testosterone levels were not affected. Epididymal spermatozoa from treated mice were in part motile, but displayed a number of abnormalities: a spectrum of irregular head shapes (from near-normal and triangular to round spatulate and oblate forms), absence of acrosomal antigens, and in a subset of sperm cells shortening and thickening of the mitochondrial sheat.

### Materials and Methods

Compounds - *N*B-DNJ was obtained from Searle/Monsanto and Oxford GlycoSciences. *N*B-DGJ was purchased from Toronto Research Chemicals. DNJ was obtained from Synergy Pharmaceuticals, Inc.

*Animals -* C57BL/6 mice were housed under standard non-sterile conditions. The mice were provided with water *ad lib.* and prior to drug administration were fed pelleted chow (expanded Rat and Mouse Chow 3, SDS Ltd.).

*Treatment of mice -* Male C57BL/6 mice were housed under standard non-sterile conditions. The mice were provided with water *ad lib.* and prior to drug administration were fed a diet of pelleted mouse chow (expanded Rat and Mouse Chow 3, SDS Ltd.) To administer the compounds of the present invention, the mice were fed a diet of powdered mouse chow (expanded Rat and Mouse Chow 1, ground, SDS Ltd.) containing either *N*B-DNJ, *N*B-DGJ, *N*N-6-MeDGJ, or DNJ. The diet and compound (both as dry solids) were mixed thoroughly, stored at room temperature, and used within 7 days of mixing.

*Mating assays -* To assess the fertility of male mice that were fed on a diet containing an iminosugar, a mating assay was used: typically six week old male C57BL/6 mice in groups of three were administered diet containing compound for five weeks (unless otherwise specified), after which each of them was caged with four untreated female C57BL/6 mice, and provided with standard pelleted chow. Females were at least 11 weeks old, and age-matched in each experiment. The male was removed from the females after 7 or 9 days, depending on the experiment; the females were monitored for vaginal mucous plugs, pregnancies, and, if any, litter sizes. To study any effect on female fertility six-week-old female C57BL/6 mice were treated with *N*B-DNJ for five weeks, after which each of them was caged with a non-treated age-matched male for four days.

*Endocrinological assays -* Following termination of mice by asphyxiation in CO₂ and cervical dislocation, blood was obtained via cardiac puncture, which was allowed to clot at room temperature (RT). Serum was obtained by centrifugation, and stored at -80°C until use. Testosterone, luteinising and follicle-stimulating hormones were measured by methods well-known in the art (Huhtaniemi, I., Nikula, H. & Rannikko, S. (1985) *J Clin Endocrinol Metab* **61,** 698-704; Haavisto, A. M., Pettersson, K., Bergendahl, M., Perheentupa, A., Roser, J. F. & Huhtaniemi, I. (1993) *Endocrinology* **132,** 1687-91; van Casteren, J. I., Schoonen, W. G. & Kloosterboer, H. J. (2000) *Biol Reprod* **62,** 886-94.)

*Fluorescence microscopy -* For immunofluorescence, freshly exised vasa deferentia were stripped and caudae epididymides were dissociated with forceps in M2 medium, and spermatozoa were allowed to disperse for 15 min. The resultant suspensions were used to prepare smears on glass slides. After drying in air for 1 hr, cells were fixed in methanol for 2 min., dried and stored at room temperature or 4 °C for one to five days. Before immunostaining smears were immersed in methanol for 5 min., dried, and wetted in PBS containing 0.5% BSA and 0.15 M glycine (PBG). Slides were stained with monoclonal antibodies, either Mab 18.6 (1:20 in PBG (Moore, H. D., Smith, C. A., Hartman, T. D. & Bye, A. P. (1987) *Gamete Res* **17**, 245-9)), anti-sp56 (1:67 in PBG, clone 7C5, QED Bioscience (Cheng, A., Le, T., Palacios, M., Bookbinder, L. H., Wassarman, P. M., Suzuki, F. & Bleil, J. D. (1994) *J Cell Biol* **125,** 867-78), and goat anti-mouse immunoglobulins γ and light chains-specific fluorescein isothiocyanate-conjugate (Ortho) according to van Dongen *et a*/*.* (van Dongen, J. M., Willemsen, R., Ginns, E. I., Sips, H. j., Tager, J. M., Barranger, J. A. & Reuser, A. J. (1985) *Eur J Cell Biol* **39,** 179-189.) Following immunostaining, nuclei were stained for 2 min in 5 µg/ml Hoechst 33342 (Molecular Probes); slides were rinsed in distilled water, and mounted with Vectashield (Vector). Fluorescent probes were examined using an Olympus BX50 microscope equipped with a Princeton Instruments CCD camera. Images were aquired, false-coloured, and overlaid with Metamorph v3.5 software, and further processed with Adobe Photoshop. For quantitation of percentages of cells stained with fluorescent reagents at least 100 cells were observed with a Zeiss Axioplan microscope.

*Histology and Scanning electronmicroscopy -* Testes were obtained from mice perfused with 4% paraformaldehyde, 1% gluteraldehyde, kept in the fixative for two days, and transferred to 70% ethanol. Tissues were post-fixed with 2% osmium tetroxide, embedded in araldite, sectioned at 1 µm, and stained with Toluidine Blue. Sections were examined with a Zeiss Axioskop II, and images were acquired with an Axiocam camera, operated by Axiovision 3.0 software.

For scanning electron microscopy, dried smears of murine spermatozoa were prepared as described above. Cells were fixed for 20 min in 4% paraformaldehyde in PBS, transferred to 70, 80 and 95% ethanol, critical-point dried in a Polaron CPD7501 instrument, gold-coated using a Nanotech Semprep2, and examined in a Hitachi S800 FEG-SEM at 3kV. For transmission electronmicroscopy epididymal spermatozoa were collected in 1 mg/ml cold water soluble polyvinylalcohol in PBS, and spun for 10 min. at 600xg. Pelleted cells were fixed overnight at 4 °C with 1 % glutaraldehyde, 0.2% picric acid in 0.2 M cacodylic acid pH 7.4, washed once in 0.1 M cacodylic acid pH 7.4, and stored in 70% ethanol. Cells were postfixed with 2% osmium tetroxide, embedded in araldite, sectioned at 70 nm, stained with uranyl acetate/lead citrate, and examined with a JEOL JEM-100CX microscope at 80 kV.

*Motility analysis.* Mice were terminated by asphyxiation in CO2 and cervical dislocation, and caudae epididymides were removed with vasa deferentia. Spermatozoa were collected in pre-warmed M2 medium (Sigma) by stripping the vasa deferentia and by applying mild pressure to the caudae epididymides; the cells were incubated at 37 °C for 1 hour, and videographed using a Leica DM IRB microscope fitted with a JVC TK-1281 camera. Computer-assisted sperm motility analysis was performed using a Hobson Sperm Tracker (Hobson Vision Ltd., UK) at 50 frames per second. Motility parameters of each sperm sample were measured in triplicate, collecting data from 200 sperm tracks per determination.

*Statistics.* Quantitative data from multiple experimental groups were analyzed for significance using multiple comparisons tests. One-way ANOVA with Tukey's post test or nonparametric ANOVA (Kruskal-Wallis test) with Dunn's post test were performed using GraphPad InStat version 3.0a for Macintosh (GraphPad Software).

### Example 1 - Effect of oral administration of NB-DNJ on male mice

To determine the effects of NB-DNJ on the fertility of male mice, six-week old C57BL/6 males were treated with 2400 mg/kg of *N*B-DNJ per day for increasing lengths of time with weekly increments (FIG. 1a), and then subjected to a mating assay, during which they were caged with untreated female mice for one week. FIG. 1A shows that, at the onset of the experiment, the males were fertile and able to sire normal-sized litters and that, after one or two weeks of *N*B-DNJ treatment, this capacity was maintained. The mice were infertile after at least three weeks of drug consumption (FIG. 1A). When male mice were taken off the drug after five weeks of treatment, they regained fertility in the fourth week, and sired normal-sized litters (FIG. 1A). Treating 13 week old males at the same dosage for five weeks also led to complete sterility (data not shown). Thus, upon *N*B-DNJ treatment, male mice become infertile, and this effect was fully reversible.

### Example 2 - Dose-response

Earlier work has shown that the in vivo reduction in GSL levels caused by *N*B-DNJ is dose-dependent; for example, doses ranging from 600 to 2400 mg/kg per day result in a 10 to 70% reduction of ganglioside GM1 levels on the surface of mouse splenocytes, respectively (Platt, et al, (1997) J Biol Chem 272, 19365-72). Having established that male mice become infertile when treated with 2400 mg/kg per day of *N*B-DNJ (Figs. 1A and 1B), they were exposed to progressively reduced doses of the compound to determine the lowest dose at which it causes infertility. The results are shown in Figures 2A and 2B. While 1 mg/kg per day did not affect the males, 5 mg/kg per day reduced their fertility (Fig. 2A) and the litter size (Fig. 2B), and 15 mg/kg per day rendered them completely infertile. Thus, the mouse male reproductive system is exceptionally sensitive to *N*B-DNJ. The drug-induced loss of fertility was not caused by a change in mating behavior, as the frequency of post-coital vaginal mucous plugs was not affected by *N*B-DNJ treatment (FIG. 2B).

### Example 3 - Fertility of Female Mice

In contrast to male mice, *N*B-DNJ treatment at 1200 mg/kg/day did not cause female mice to be infertile. Following a four-day mating period with untreated males, 100% of treated (n = 5) as well as of non-treated females (n = 4) became pregnant; these mice had 6.2 ± 2.7 and 7.8 ± 1.0 pups per litter, respectively.

*N*B-DNJ was administered at 2400 mg/kg day for 12 weeks to 15 week-old female mice of strain 129. After this, the drug was withdrawn and the females were caged with untreated males of strain 129. All females quickly became pregnant and had litters of an average 6 pups. The mice in this experiment were heterozygous for a disruption in the gene encoding ceramide galactosyltransferase. These mice are phenotypically indistinguishable from wild-type 129 mice and reproduce normally (Coetzee *et al.,* (1996), Cell, **86**:209-219).

### Example 4 - Endocrinology

The serum levels of testosterone, follicle-stimulating hormone (FSH), luteinizing hormone (LH), and testicular testosterone were determined in *N*B-DNJ-treated mice. FIGs. 3A - 3D show that 15, 150 or 1 200 mg/kg/day of the compound did not change these endocrinological parameters. In contrast, 2400 mg/kg/day caused a significant reduction (p < 0.001) in all of these hormone levels. Thus, the reproductive endocrinology of the imino sugar-treated males is distinct from that of the complex ganglioside-deficient animals. Furthermore, these results indicate that the *N*B-DNJ-induced infertility (requiring at least 15 mg/kg/day) is not mediated by a depression of reproductive hormones.

### Example 5 - Gonad Weights

In this study, low-dose *N*B-DNJ treatment (15-150 mg/kg/day) did not affect body or gonad weights. At 1200 and 2400 mg/kg/day weights of testes and epididymal compartments were decreased proportionally to body weight. The only exception was seen at the highest dose, where the combined cauda epididymis/vas deferens weight was reduced further (p < 0.05) than the whole body weight (43 ± 7% vs. 60 ± 5% of control, respectively, (n= 10)). Thus, gonadal weight loss is an effect of a high-dosage *N*B-DNJ regimen, and unrelated to induction of male infertility. Furthermore, the loss of fertility caused by the drug was not associated with a reduction in epididymal sperm count.

### Example 7 - Testis Histology

In the seminiferous tubules of *N*B-DNJ-treated animals a more extensive vacuolization was observed, at a higher frequency, than in controls (FIGS. 4 A and B). In addition, following administration of drug, the nuclei of mature spermatids displayed an aberrant, heterogeneous morphology. The inset in FIG. 4B shows an example of such step 13 spermatids: they often had indentations, and were much less elongated than normal step 13 spermatids (FIG. 4A, inset) (for classification of spermatids and testicular staging see Russell *et al* (1990), Histological and Histopathological Evaluation of the Testis, Cache River Press, Clearwater, FL, USA).

### Example 8 - Effects on morphology of spermatozoa

The spermatozoa of mice treated with *N*B-DNJ were examined to see if they had an altered morphology.

The nucleus of a normal murine epididymal spermatozoon is laterally flattened and has a typically curved, falciform shape. Overlying the nucleus is the acrosome, a relatively large membrane-delineated organelle, that contains various hydrolytic enzymes, and can be regarded as a specialized regulated secretory vesicle; the acrosome is essential for fertilisation (reviewed in Yanagimachi, R. (1994) in The Physiology of Reproduction, eds. Knobil, E. and Neill, J. D. (Raven Press, New York), pp. 189 - 317). In agreement with the histological observations of the testis (see Example 7 above), cauda epididymal sperm cells from *N*B-DNJ-treated mice had various abnormally shaped nuclei: they were either near-normal, triangular, oblate, or elongate, the majority being spatulate (FIGS. 4D, 4E and 4F) (morphological categories as described by Hollander *et al.* (1960) *Fertil Steril* **11**, 316 - 324). Some of these nuclei had a lateral invagination close to the anterior tip. Spermatozoa derived from the caput epididymis were similarly affected. The percentage of caudal spermatozoa with (near)-normal nuclei decreased dramatically with increasing *N*B-DNJ dosage, falling below 6% at the minimal dose to achieve complete infertility (FIG. 2C). Curiously, normal nuclear morphology was significantly more often (p < 0.01) observed at 1200 mg/kg/day than at 50-150 mg/kg/day of *N*B-DNJ (FIG. 2C).

To further characterize these spermatozoa, indirect immunofluorescence was employed with monoclonal antibodies Mab18.6 and anti-sp56, which recognize distinct acrosomal antigens (Moore *et al., supra*; Cheng *et al.* (1994) *J Cell Biol* **125**, 867 - 878). Only a small fraction of the spermatozoa from *N*B-DNJ-treated mice stained with Mab18.6 (FIG. 4D) or anti-sp56 (data not shown), decreasing with drug dose to below 10% at 15 mg/kg/day (FIG. 2D). Again 1200 mg/kg/day was exceptional: antibody staining at this dose was more frequent than at 15-150 mg/kg/day (FIG. 2D). However, many of the Mab 18.6-positive sperm cells from drug-treated mice did not show the normal crescent-shaped acrosomal fluorescence, but displayed an irregular staining pattern (FIGS. 4E and 4F). In fact, among the Mab 18.6-positive cells the incidence of (near)-normal acrosomal staining was equally low from 15 to 1200 mg/kg/day, on average 31 ± 10% (n = 3). With anti-sp56 similar results were obtained. Sperm samples from mice administered with 2400 mg/kg/day had similar characteristics as those from mice treated with 1200 mg/kg/day (data not shown). Finally, also at the ultrastructural level, no typical acrosomal elements were seen covering the nuclei of epididymal spermatozoa (FIGS. 5D, 5E and 5F).

A segment (midpiece) of the tail of a normal murine spermatozoon close to the sperm head carries the mitochondria, which are arranged in two parallel helices, and form a single-layered sheath around the core elements of the tail (reviewed in (Eddy *et al., supra*). These mitochondria are very uniform in shape and width. *N*B-DNJ treatment induced a number of abnormalities in the tails and mitochondria of epididymal spermatozoa. Firstly, in a subset of the spermatozoa from *N*B-DNJ-treated mice, the mitochondria were found in a much shorter and wider arrangement just behind or adjacent to the sperm head (FIGS. 5B and 5F). The fibrous tail core structures (outer dense fibers/axoneme) of this type of cell were devoid of a mitochondrial investment over a large part of the midpiece, from the posterior end of the altered mitochondrial sheath to the beginning of the next tail segment, the principal piece. These shortened sheaths were highly disorganized: the mitochondria were irregularly arranged and stacked on top of each other; they varied widely in size and shape (FIGS. 5B and 5F).

The tail of a normal murine spermatozoon extends away from the head, and can be mildly curved. However, in a fraction of epididymal spermatozoa from *N*B-DNJ-treated mice, the proximal part of the tail was wound around the sperm head or nucleus, from a half or single turn (FIG. 5E) to several full loops (FIG. 5D). Coiling of the tail in this fashion positioned the section of the tail carrying the mitochondrial sheath at least in part in the sperm head, adjacent to the nucleus (FIG. 5E). Finally, drug treatment rendered spermatozoal nuclei less condensed, displaying small electronlucent foci (FIGS. 5D, 5E and 5F), which were much less frequent in control cells (FIG. 5C).

Thus, *N*B-DNJ treatment lead to nuclear dysmorphia of spermatozoa, induces acrosomal abnormalities, and causes disorganization of the tail, including the mitochondrial sheath. A relatively low drug dose induced a very high proportion of abnormal sperm cells. Surprisingly, at the highest doses used, this effect was partially reversed. The galactose analogue *N*B-DGJ, used at 1200 mg/kg/day, induced a similar range of morphological aberrations as seen with *N*B-DNJ (data not shown).

### Example 9 - Sperm Motility

To assess whether the structural defects of epididymal spermatozoa from *N*B-DNJ-treated mice had any functional consequences, we performed computer-assisted sperm motility analysis. At 15 mg/kg/day the motile percentage was significantly below normal (p < 0.05), as were curvilinear velocity and straight line velocity (p<0.001) (Table 1).

**Table 1. Sperm motility.**

| *N*B-DNJ (mg/kg/day) | % motile | VCL (µm/sec) | VSL (µm/sec) |
|---|---|---|---|
| 0 | 74±12 | 182±19 | 52±10 |
| 15 | 26±16 | 74±31 | 27±9 |
| 150 | 15±6 | 89±31 | 27±8 |
| 1200 | 17±7 | 49±17 | 18±9 |
| 2400 | 9±2 | 28±21 | 9±5 |

| | | | |
|---|---|---|---|
| VCL, curvilinear velocity; VSL, straight line velocity. The data are presented as the mean ± SD; n ≥ 3. | | | |

Moreover, at 2400 mg/kg/day both velocities were significantly lower than at 150 mg/kg/day (p<0.001) (Table 1). Thus, ingestion of *N*B-DNJ leads to a severely diminished sperm motility, which is progressive with drug dose.

### Example 10 - Effect of NN-DGJ on Male Mice

In this example, eight mice (n = 8) were treated with 250 mg/kg/day of *N*N-DGJ over a 40 day period. As shown in FIGURE 6A, *N*N-DGJ did not affect the body weight of the treated mice relative to that of untreated mice.

Liver glycogen levels were measured in untreated and *N*N-DGJ-treated mice (FIG. 6B), who were either provided food or starved. *N*N-DGJ did not inhibit the breakdown of liver glycogen during starvation.

*N*N-DGJ did not affect the weight of thymus and spleen weights in treated mice. As shown in FIGURE 6C, thymus and spleen weights between *N*N-DGJ-treated and untreated mice were virtually identical.

*N*N-DGJ exhibited a potent and reversible effect on the fertility of male mice in an analogous experiment involving four treated mice and three untreated mice. As shown in FIGURE 6D, administration of *N*N-DGJ completely suppressed the fertility of the mice after five weeks of treatment, but at six weeks following withdrawal of *N*N-DGJ, the mice fully regained fertility. This reversible effect was also manifested in litter size: six weeks after withdraw of *N*N-DGJ, the litter size of females mater with treated males was almost the same as the litter size of females mated with untreated males (FIGURE 6E).

### Example 11 - Effect of NN-6-MeDGJ on Male Mice Fertility

Six male mice were treated orally at either 15 (low) or 250 mg/kg/day (high) *N*N-6-MeDGJ (FIGS. 8A and 8B). Additionally, *N*N-6-MeDGJ was administered at 1 5 mg/kg/day through a subcutaneously implanted releasing device (Alzet minipump; "pump"). After treatment for five weeks, the fertility of the male mice was evaluated in a mating assay as described above. FIGURES 8A and 8B show that at the high dosage, male mice became infertile; low dose-treated mice exhibited reduced fertility; and the fertility of the mice that were administered *N*N-6-MeDGJ via pump was lower than that of the orally fed low-dose mice. After five weeks of withdrawing *N*N-6-MeDGJ, the fertility of the high-dose mice was the same as that of the control mice. Thus, the fertility of male mice had been completely suppressed, and then completely regained.

The effects of *N*N-6-MeDGJ on male mice bodies and behavior did not differ substantially from those of untreated mice. Eight mice were fed *N*N-6-MeDGJ at 250 mg/kg/day over a 40 day period, during which the body weights of the *N*N-6-MeDGJ-treated mice were the same as those of untreated mice (FIG. 7A). Additionally, *N*N-6-MeDGJ did not affect the gastrointestinal tract, thymus, and spleen weights of male mice (FIG. 7C). Finally, spontaneous behavior in *N*N-6-MeDGJ-treated mice were compared to that in untreated mice. As shown in FIGURE 7B, *N*N-6-MeDGJ did not affect the movement of treated mice.

## Claims

1. A method of reversibly rendering a fertile male mammal infertile, comprising the steps of:
(a) administering to said mammal a pharmaceutical composition comprising one or more pharmaceutically acceptable excipients and a therapeutically effective amount of a compound of formula **I,** or a stereoisomer, pharmaceutically acceptable salt, or solvate thereof: 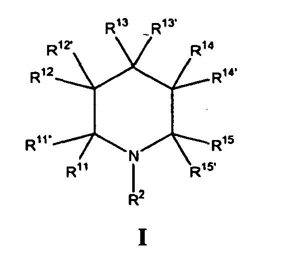 wherein each of R¹¹, R^{11'}, R¹², R^{12'}, R¹³, R^{13'}, R¹⁴, R^{14'}, R¹⁵ and R^{15'} is selected, independently from each other, from the group consisting of -H; -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy; aryloxy; aralkoxy; -(alkylene)oxy(alkyl); -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; -NHOH; aryl, and heteroaryl;
wherein each alkyl, alkenyl, alkynyl, aryl, and heteroaryl moiety may be optionally substituted with one or more groups independently selected from the group consisting of -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy, aryloxy; aralkoxy; -(alkylene)oxy(alkyl); -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; and -NHOH;
and wherein at least two of R¹¹, R^{11'}, R¹², R^{12'}, R¹³, R^{13'}, R¹⁴, R^{14'}, R¹⁵, and R^{15'} are selected from the group consisting of -CH₃, -CH₂OH, and -OH.
R² is a substituent selected from the group consisting of linear or branched C₁₋₁₈ alkyl, C₂₋₁₈ alkenyl and alkynyl; and aralkyl,
wherein each alkyl, alkenyl, alkynyl, and aryl moiety may be optionally substituted with one or more groups independently selected from the group consisting of -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy, aryloxy; aralkoxy; -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; and -NHOH;
with the proviso that the compounds of formula I or their pharmaceutically acceptable salt thereof do not have the following formula 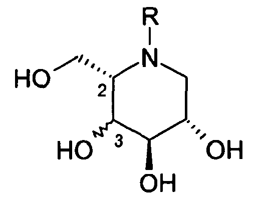 wherein R is C₁₋₁₆ straight or branched-chain alkyl, optionally substituted by C₃₋₇ cycloalkyl, and optionally interrupted by -O- the oxygen being separated from the ring nitrogen by at least two carbon atoms, or C₁₋₁₀ alkylaryl where aryl is phenyl, pyridyl, thienyl or furyl wherein phenyl is optionally substituted by one or more substituents selected from F, Cl, Br, CF₃, OCF₃, OR¹, and C₁₋₆ straight or branched-chain alkyl; and
R¹ is hydrogen, or C₁₋₆ straight or branched-chain alkyl;
said composition being administered in an amount and for a time sufficient to render said male mammal infertile; and
(b) cease administering said composition to said mammal, whereby said mammal is rendered fertile.

2. The method according to claim 1 wherein at least one of R¹¹, R¹¹, R¹², R^{12'}, R¹³, R^{13'}, R¹⁴, R^{14'}, R¹⁵, and R^{15'} is -CH₂OH.

3. The method according to claim 1 or 2 wherein at least one of R¹¹, R^{11'}, R¹², R^{12'}, R¹³, R^{13'}, R¹⁴, R^{14'}, R¹⁵, and R^{15'} is -OH.

4. The method according to claims 1 to 3 wherein R² is a linear or branched C₁₋₁₈ alkyl group.

5. The method according to claim 4 wherein R² is a linear or branched C₄₋₉ alkyl group.

6. The method according to claims 1 to 5 wherein the compound is one selected from the group consisting of: 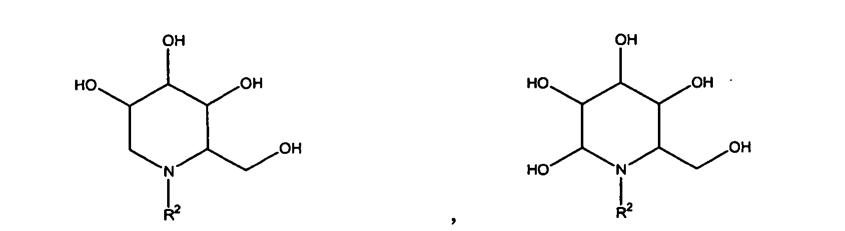 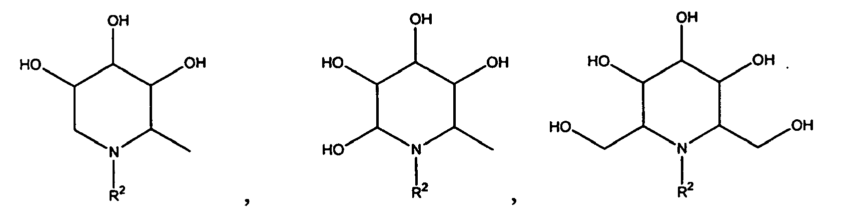 and stereoisomers thereof.

7. The method according to claim 6 wherein the compound is one selected from the group consisting of compounds set forth in the following table:

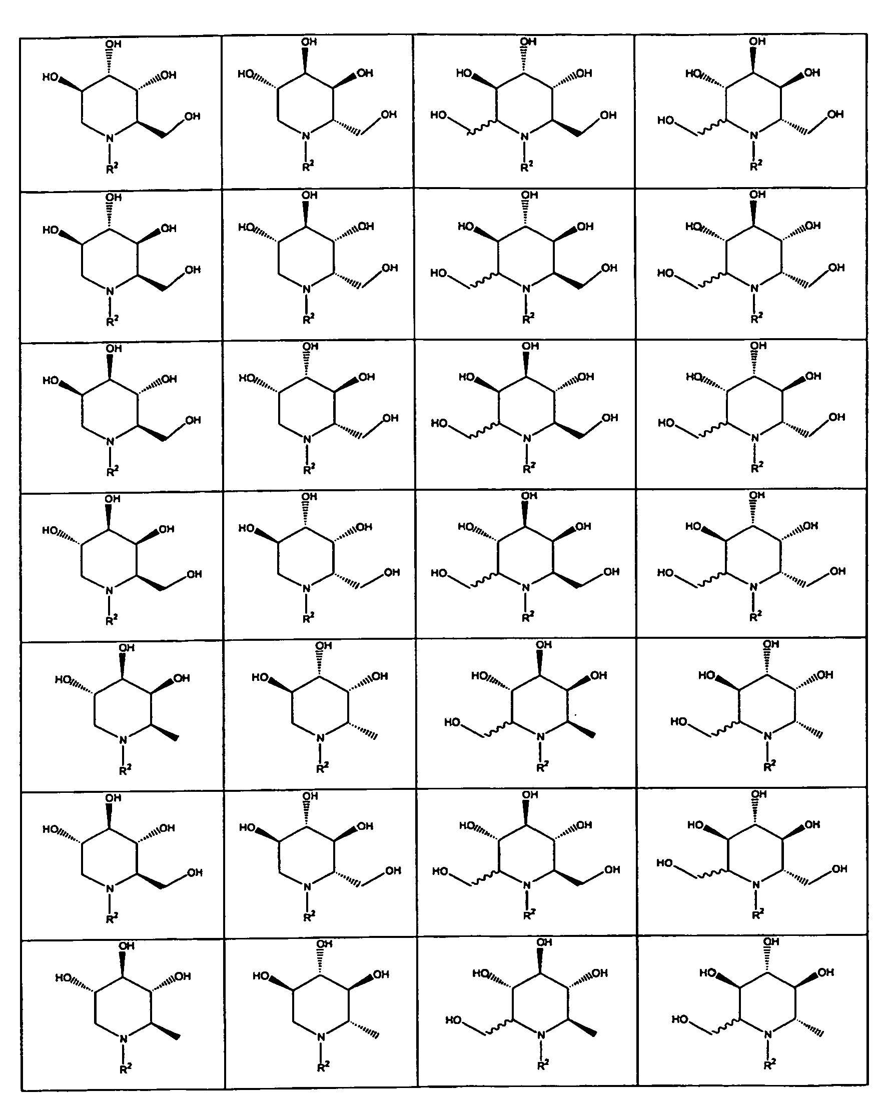

8. The method according to claim 7 wherein R² is a linear or branched C₁₋₁₈ alkyl group optionally substituted with C₁₋₆ alkoxy.

9. The method according to claim 8, wherein the compound is one selected from the group consisting of: 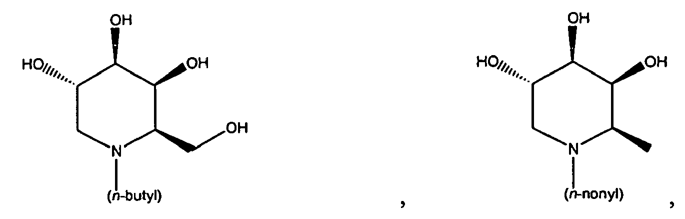 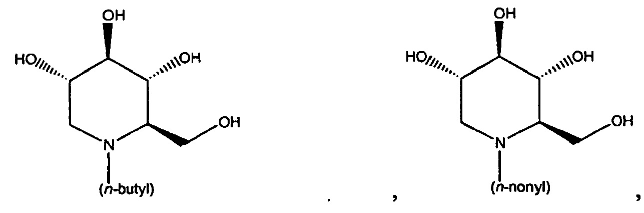 and

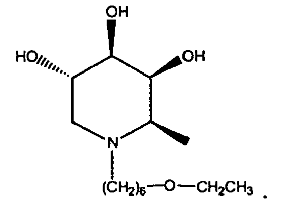

10. The method according to claims 1 to 9, further comprising the steps of sequentially repeating steps (a) and (b).

11. A method of reversibly rendering a fertile male mammal infertile, comprising the steps of:
(a) administering to said mammal a pharmaceutical composition comprising one or more pharmaceutically acceptable excipients and a therapeutically effective amount of a compound of formula **I**, or a stereoisomer, pharmaceutically acceptable salt, or solvate thereof: 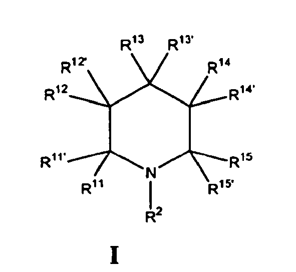 wherein each of R¹¹, R^{11'}, R¹², R^{12'} R¹³, R^{13'}, R¹⁴, R^{14'}, R¹⁵ and R^{15'} is selected, independently from each other, from the group consisting of -H; -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy; aryloxy; aralkoxy; -(alkylene)oxy(alkyl); -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; -NHOH; aryl, and heteroaryl;
wherein each alkyl, alkenyl, alkynyl, aryl, and heteroaryl moiety may be optionally substituted with one or more groups independently selected from the group consisting of -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy, aryloxy; aralkoxy; -(alkylene)oxy(alkyl); -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; and -NHOH;
and wherein at least two of R¹¹, R^{11'}, R¹², R^{12'}, R¹³, R^{13'}, R¹⁴, R^{14'}, R¹⁵, and R^{15'} are selected from the group consisting of -CH₃, -CH₂OH, and -OH.
R² is a substituent selected from the group consisting of linear or branched C₁₋₁₈ alkyl, C₂₋₁₈ alkenyl and alkynyl; and aralkyl,
wherein each alkyl, alkenyl, alkynyl, and aryl moiety may be optionally substituted with one or more groups independently selected from the group consisting of -OH; -F; -Cl; -Br; -I; -NH₂; alkyl- and dialkylamino; linear or branched C₁₋₆ alkyl, C₂₋₆ alkenyl and alkynyl; aralkyl; linear or branched C₁₋₆ alkoxy, aryloxy; aralkoxy; -CN, -NO₂, -COOH, -COO(alkyl); -COO(aryl); -C(O)NH(C₁₋₆ alkyl); -C(O)NH(aryl); sulfonyl; (C₁₋₆ alkyl)sulfonyl; arylsulfonyl; sulfamoyl, (C₁₋₆ alkyl)sulfamoyl; (C₁₋₆ alkyl)thio; (C₁₋₆ alkyl)sulfonamide; arylsulfonamide; -NHNH₂; and -NHOH;
with the proviso that the compounds of formula I or their pharmaceutically acceptable salt thereof do not have the following formula 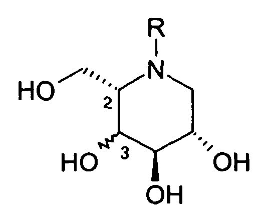 wherein R is C₁₋₁₆ straight or branched-chain alkyl, optionally substituted by C₃₋₇ cycloalkyl, and optionally interrupted by -O- the oxygen being separated from the ring nitrogen by at least two carbon atoms, or C₁₋₁₀ alkylaryl where aryl is phenyl, pyridyl, thienyl or furyl wherein phenyl is optionally substituted by one or more substituents selected from F, Cl, Br, CF₃, OCF₃, OR¹, and C₁₋₆ straight or branched-chain alkyl; and
R¹ is hydrogen, or C₁₋₆ straight or branched-chain alkyl;
said composition being administered in an amount and for a time sufficient to render said male mammal infertile; and
(b) cease administering said composition to said mammal, whereby said mammal is rendered fertile; and
(c) repeating steps (a) and (b) one or more times.

12. The method according to claim 11 wherein at least one of R¹¹, R^{11'}, R¹², R^{12'}, R¹³, R^{13'}, R¹⁴, R^{14'}, R¹⁵, and R^{15'} is -CH₂OH.

13. The method according to claim 11 or 12 wherein at least one of R¹¹, R^{11'}, R¹² R^{12'}, R¹³, R^{13'}, R¹⁴, R^{14'}, R¹⁵ and R^{15'} is -OH.

14. The method according to claims 11 to 13 wherein R² is a linear or branched C₁₋₁₈ alkyl group.

15. The method according to claim 14 wherein R² is a linear or branched C₄₋₉ alkyl group.

16. The method according to claims 11 to 15 wherein the compound is one selected from the group consisting of: 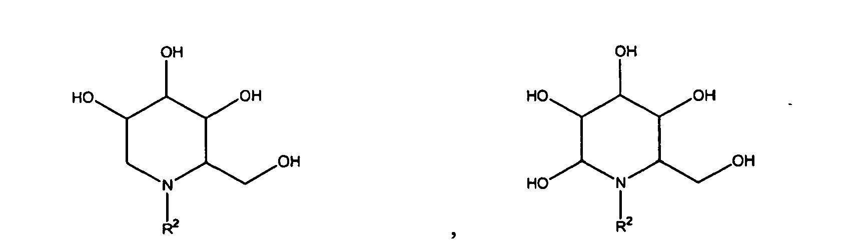 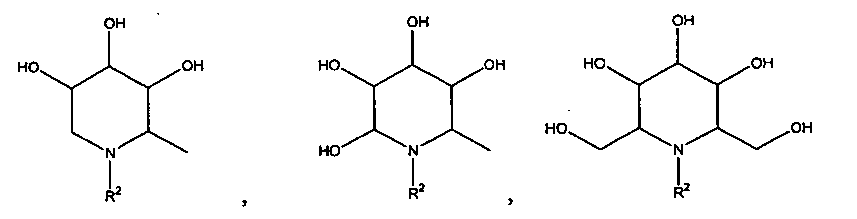 and stereoisomers thereof.

17. The method according to claim 16 wherein the compound is one selected from the group consisting of compounds set forth in the following table:

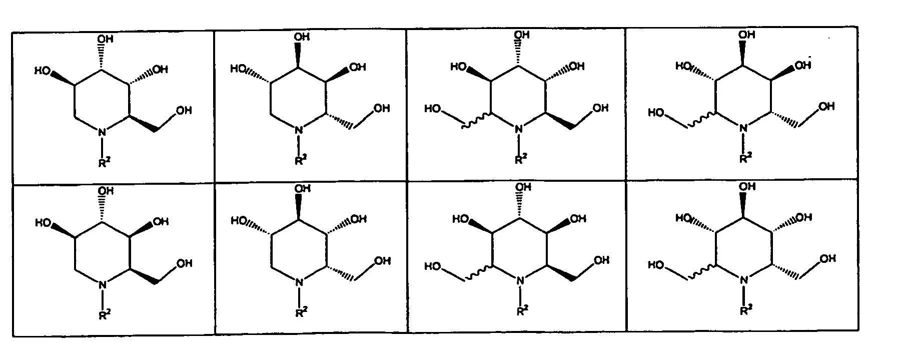

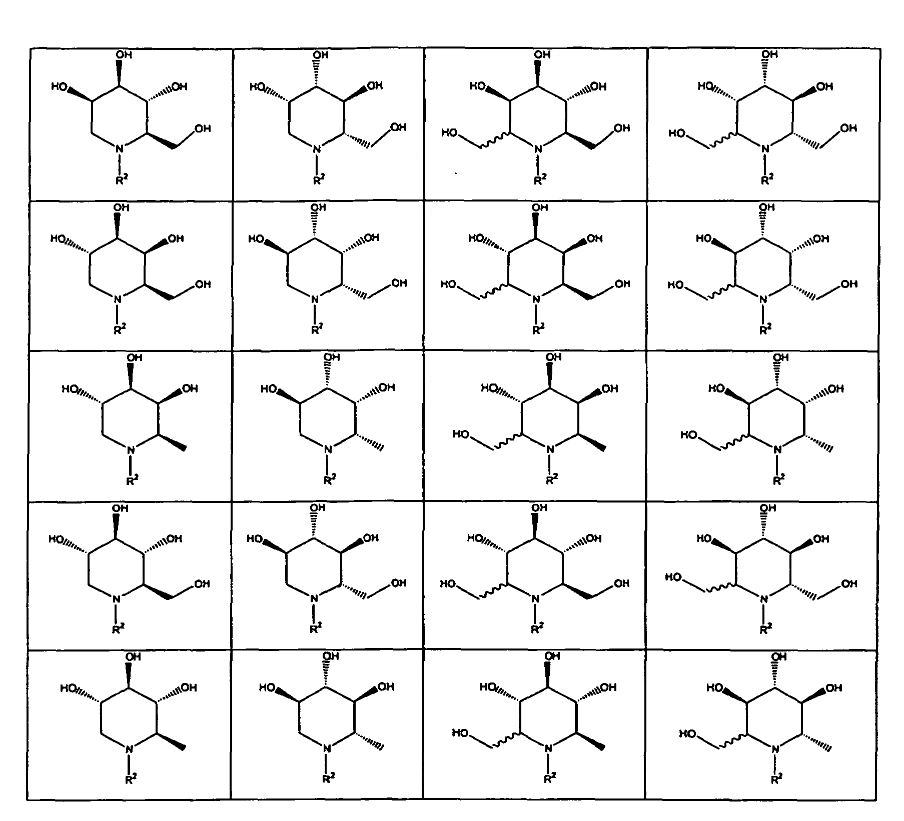

18. The method according to claim 17 wherein R² is a linear or branched C₁₋₁₈ alkyl group optionally substituted with C₁₋₆ alkoxy.

19. The method according to claim 18, wherein the compound is one selected from the group consisting of: 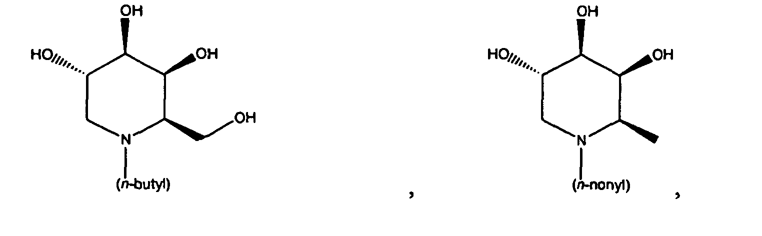 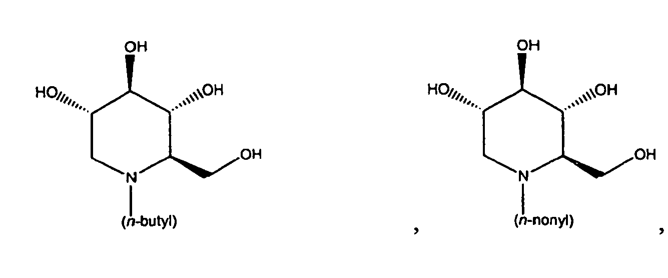 and

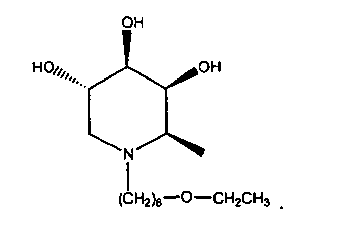

20. The method according to claims 11 to 19, further comprising the steps of sequentially repeating steps (a), (b), and (c).

21. The method according to claims 1 to 20 wherein said mammal is human.

22. The method according to claims 1 to 20 wherein said mammal is a companion mammal.

23. The method according to claim 22 wherein said mammal is one selected from the group consisting of dogs, cats, and horses.

24. The method according to claims 1 to 20 wherein said mammal is an ungulate.

25. The method according to claim 24 wherein said mammal is one selected from the group consisting of cattle, sheep, goats, water buffalo, camels, and pigs.

26. The method according to claims 1 to 20 wherein said mammal is one selected from apes, monkeys, llamas, rodents, and rabbits.

## Patentansprüche

1. Verfahren, um einen fruchtbaren männlichen Säuger reversibel unfruchtbar zu machen, welches die Schritte umfasst:
(a) Verabreichen einer pharmazeutischen Zusammensetzung an den Säuger, welche einen oder mehrere pharmazeutisch annehmbare Hilfsstoffe und eine therapeutisch wirksame Menge einer Verbindung der Formel **I** oder eines Stereoisomers, pharmazeutisch annehmbaren Salzes oder Solvats derselben umfasst: 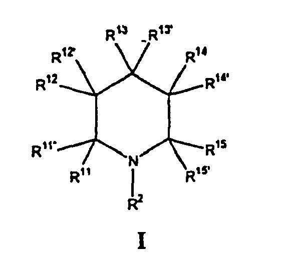 worin jedes von R¹¹, R^{11'}, R¹², R^{12'}, R¹³, R^{13'}, R¹⁴, R^{14'}, R¹⁵ und R^{15'} unabhängig voneinander ausgewählt ist aus der Gruppe bestehend aus -H; -OH; -F; -Cl; -Br; -I; -NH₂; Alkyl- und Dialkylamino; linearem oder verzweigtem C₁₋₆-Alkyl, C₂₋₆-Alkenyl und -Alkinyl; Aralkyl; linearem oder verzweigtem C₁₋₆-Alkoxy; Aryloxy; Aralkoxy; -(Alkylen)oxy(alkyl); -CN, -NO₂, -COOH, -COO(Alkyl); -COO(Aryl); -C(O)NH(C₁₋₆-Alkyl); -C(O)NH(Aryl); Sulfonyl; (C₁₋₆-Alkyl)sulfonyl; Arylsulfonyl; Sulfamoyl, (C₁₋₆-Alkyl)sulfamoyl; (C₁₋₆-Alkyl)thio; (C₁₋₆₋Alkyl)sulfonamid; Arylsulfonamid; -NHNH₂; -NHOH; Aryl und Heteroaryl;
wobei jede Alkyl-, Alkenyl-, Alkinyl-, Aryl- und Heteroaryl-Einheit gegebenenfalls mit einer oder mehreren Gruppen substituiert sein kann, die unabhängig ausgewählt sind aus der Gruppe bestehend aus -OH; -F; -Cl; -Br; -I; -NH₂; Alkyl- und Dialkylamino; linearem oder verzweigtem C₁₋₆-Alkyl, C₂₋₆-Alkenyl und -Alkinyl; Aralkyl; linearem oder verzweigtem C₁₋₆-Alkoxy, Aryloxy; Aralkoxy; -(Alkylen)oxy(alkyl); -CN; -NO₂, -COOH, -COO(Alkyl); -COO(Aryl); -C(O)NH(C₁₋₆-Alkyl); -C(O)NH(Aryl); Sulfonyl; (C₁₋₆-Alkyl)sulfonyl; Arylsulfonyl; Sulfamoyl, (C₁₋₆-Alkyl)sulfamoyl; (C₁₋₆Alkyl)thio; (C₁₋₆-Alkyl)sulfonamid; Arylsulfonamid; -NHNH₂; und -NHOH;
und wobei mindestens zwei von R¹¹, R^{11'}, R¹², R^{12'}, R¹³, R^{13'}, R¹⁴, R^{14'}, R¹⁵ und R^{15'} ausgewählt sind aus der Gruppe bestehend aus -CH₃, -CH₂OH und -OH;
R² ein Substituent ist, der ausgewählt ist aus der Gruppe bestehend aus linearem oder verzweigtem C₁₋₁₈-Alkyl, C₂₋₁₈-Alkenyl und -Alkinyl; und Aralkyl,
wobei jede Alkyl-, Alkenyl-, Alkinyl- und Aryl-Einheit gegebenenfalls mit einer oder mehreren Gruppen substituiert sein kann, die unabhängig ausgewählt sind aus der Gruppe bestehend aus -OH; -F; -Cl; -Br; -I; -NH₂; Alkyl- und Dialkylamino; linearem oder verzweigtem C₁₋₆-Alkyl, C₂₋₆-Alkenyl und -Alkinyl; Aralkyl; linearem oder verzweigtem C₁₋₆-Alkoxy, Aryloxy; Aralkoxy; -CN; -NO₂, -COOH, -COO(Alkyl); -COO(Aryl); -C(O)NH(C₁₋₆-Alkyl); -C(O)NH(Aryl); Sulfonyl; (C₁₋₆-Alkyl)sulfonyl; Arylsulfonyl; Sulfamoyl, (C₁₋₆₋Alkyl)sulfamoyl; (C₁₋₆-Alkyl)thio; (C₁₋₆-Alkyl)sulfonamid; Arylsulfonamid; -NHNH₂; und -NHOH;
mit der Maßgabe, dass die Verbindungen der Formel I oder deren pharmazeutisch annehmbares Salz nicht die folgende Formel 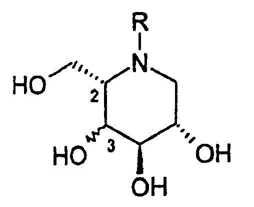 aufweisen, in der R für gerades oder verzweigtkettiges C₁₋₁₆-Alkyl, das gegebenenfalls mit C₃₋₇-Cycloalkyl substituiert ist und gegebenenfalls durch -O- unterbrochen ist, wobei der Sauerstoff von dem Ring-Stickstoff durch mindestens zwei Kohlenstoffatome getrennt ist, oder C₁₋₁₀-Alkylaryl steht, worin Aryl für Phenyl, Pyridyl, Thienyl oder Furyl steht, wobei Phenyl gegebenenfalls mit einem oder mehreren Substituenten substituiert ist, die ausgewählt sind aus F, Cl, Br, CF₃, OCF₃, OR¹ und geradem oder verzweigtkettigem C₁₋₆₋Alkyl; und
R¹ Wasserstoff oder gerades oder verzweigtkettiges C₁₋₆-Alkyl ist;
wobei die Zusammensetzung in einer Menge und über eine Zeit verabreicht wird, die ausreichen, um den männlichen Säuger unfruchtbar zu machen; und
(b) Aufhören mit der Verabreichung der Zusammensetzung an den Säuger, wodurch der Säuger fruchtbar gemacht wird.

2. Verfahren nach Anspruch 1, in dem mindestens eines von R¹¹, R^{11'}, R¹², R^{12'}, R¹³, R^{13'}, R¹⁴, R^{14'}, R¹⁵ und R^{15'} für -CH₂OH steht.

3. Verfahren nach Anspruch 1 oder 2, in dem mindestens eines von R¹¹, R^{11'}, R¹², R^{12'}, R¹³, R^{13'}, R¹⁴, R^{14'}, R¹⁵ und R^{15'} für -OH steht.

4. Verfahren nach den Ansprüchen 1 bis 3, in dem R² eine lineare oder verzweigte C₁₋₁₈-Alkylgruppe ist.

5. Verfahren nach Anspruch 4, in dem R² eine lineare oder verzweigte C₄₋₉₋Alkylgruppe ist.

6. Verfahren nach den Ansprüchen 1 bis 5, in dem die Verbindung eine ist, die ausgewählt ist aus der Gruppe bestehend aus: 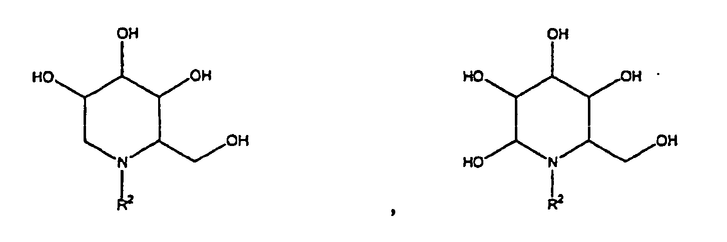 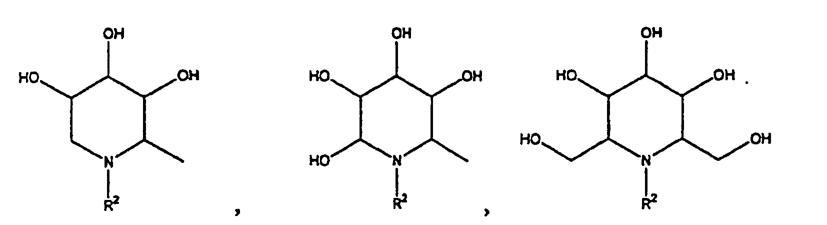 und deren Stereoisomeren.

7. Verfahren nach Anspruch 6, in dem die Verbindung eine ist, die ausgewählt ist aus der Gruppe bestehend aus Verbindungen, die in der folgenden Tabelle aufgeführt sind:

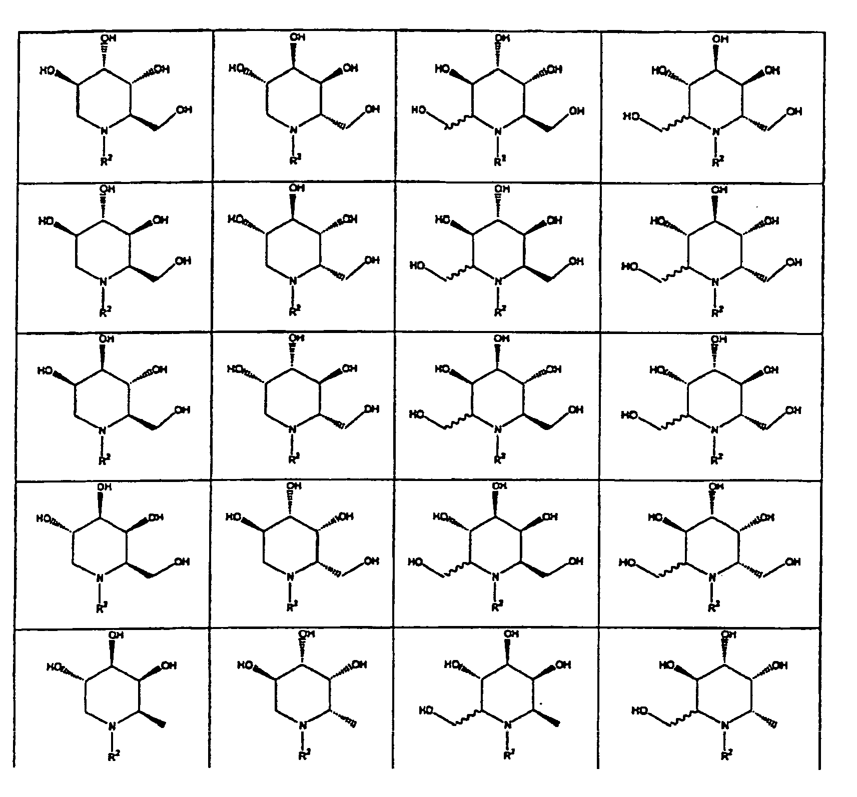

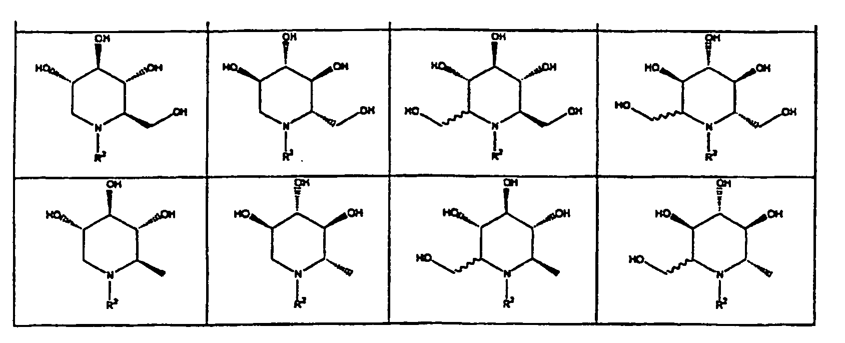

8. Verfahren nach Anspruch 7, in dem R² eine lineare oder verzweigte C₁₋₁₈₋Alkylgruppe ist, die gegebenenfalls mit C₁₋₆-Alkoxy substituiert ist.

9. Verfahren nach Anspruch 8, in dem die Verbindung eine ist, die ausgewählt ist aus der Gruppe bestehend aus: 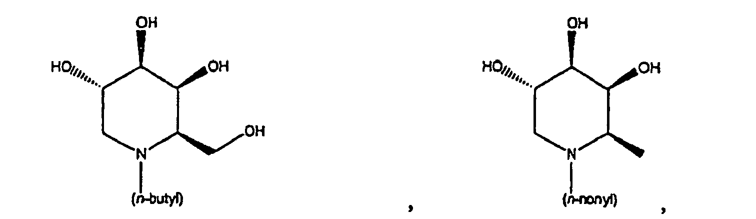 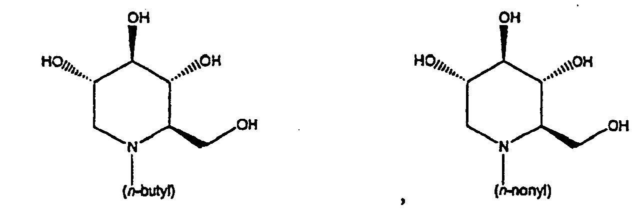 und

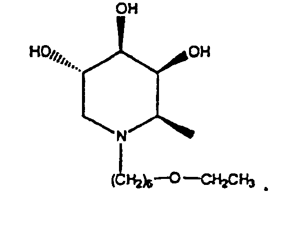

10. Verfahren nach den Ansprüchen 1 bis 9, weiter umfassend die Schritte der aufeinanderfolgenden Wiederholung der Schritte (a) und (b).

11. Verfahren, um einen fruchtbaren männlichen Säuger reversibel unfruchtbar zu machen, welches die Schritte umfasst:
(a) Verabreichen einer pharmazeutischen Zusammensetzung an den Säuger, welche einen oder mehrere pharmazeutisch annehmbare Hilfsstoffe und eine therapeutisch wirksame Menge einer Verbindung der Formel **I** oder eines Stereoisomers, pharmazeutisch annehmbaren Salzes oder Solvats derselben umfasst: 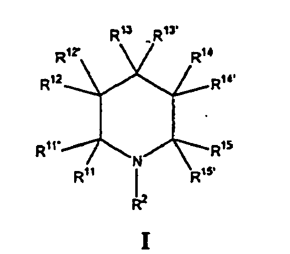 worin jedes von R¹¹, R^{11'}, R¹², R^{12'}, R¹³, R^{13'} , R¹⁴, R^{14'} , R¹⁵ und R^{15'} unabhängig voneinander ausgewählt ist aus der Gruppe bestehend aus -H; -OH; -F; -Cl; -Br; -I; -NH₂; Alkyl- und Dialkylamino; linearem oder verzweigtem C₁₋₆-Alkyl, C₂₋₆-Alkenyl und -Alkinyl; Aralkyl; linearem oder verzweigtem C₁₋₆-Alkoxy; Aryloxy, Aralkoxy; -(Alkylen)oxy(alkyl); -CN, -NO₂, -COOH, -COO(Alkyl); -COO(Aryl); -C(O)NH(C₁₋₆-Alkyl); -C(O)NH(Aryl); Sulfonyl; (C₁₋₆Alkyl)sulfonyl; Arylsulfonyl; Sulfamoyl, (C₁₋₆-Alkyl)sulfamoyl; (C₁₋₆-Alkyl)thio; (C₁₋₆₋Alkyl)sulfonamid; Arylsulfonamid; -NHNH₂; -NHOH; Aryl und Heteroaryl;
wobei jede Alkyl-, Alkenyl-, Alkinyl-, Aryl- und Heteroaryl-Einheit gegebenenfalls mit einer oder mehreren Gruppen substituiert sein kann, die unabhängig ausgewählt sind aus der Gruppe bestehend aus -OH; -F; -Cl; -Br; -I; -NH₂; Alkyl- und Dialkylamino; linearem oder verzweigtem C₁₋₆-Alkyl, C₂₋₆-Alkenyl und -Alkinyl; Aralkyl; linearem oder verzweigtem C₁₋₆-Alkoxy, Aryloxy; Aralkoxy; -(Alkylen)oxy(alkyl); -CN; -NO₂, -COOH, -COO(Alkyl); -COO(Aryl); -C(O)NH(C₁₋₆-Alkyl); -C(O)NH(Aryl); Sulfonyl; (C₁₋₆-Alkyl)sulfonyl; Arylsulfonyl; Sulfamoyl, (C₁₋₆-Alkyl)sulfamoyl; (C₁₋₆-Alkyl)thio; (C₁₋₆-Alkyl)sulfonamid; Arylsulfonamid; -NHNH₂; und -NHOH;
und wobei mindestens zwei von R¹¹, R^{11'}, R¹², R^{12'}, R¹³, R^{13'}, R¹⁴, R^{14'}, R¹⁵ und R^{15'} ausgewählt sind aus der Gruppe bestehend aus -CH₃, -CH₂OH und -OH;
R² ein Substituent ist, der ausgewählt ist aus der Gruppe bestehend aus linearem oder verzweigtem C₁₋₁₈-Alkyl, C₂₋₁₈-Alkenyl und -Alkinyl; und Aralkyl,
wobei jede Alkyl-, Alkenyl-, Alkinyl- und Aryl-Einheit gegebenenfalls mit einer oder mehreren Gruppen substituiert sein kann, die unabhängig ausgewählt sind aus der Gruppe bestehend aus -OH; -F; -Cl; -Br; -I; -NH₂; Alkyl- und Dialkylamino; linearem oder verzweigtem C₁₋₆-Alkyl, C₂₋₆-Alkenyl und -Alkinyl; Aralkyl; linearem oder verzweigtem C₁₋₆-Alkoxy, Aryloxy; Aralkoxy; -CN; -NO₂, -COOH, -COO(Alkyl); -COO(Aryl); -C(O)NH(C₁₋₆-Alkyl); -C(O)NH(Aryl); Sulfonyl; (C₁₋₆-Alkyl)sulfonyl; Arylsulfonyl; Sulfamoyl, (C₁₋₆₋Alkyl)sulfamoyl; (C₁₋₆-Alkyl)thio; (C₁₋₆-Alkyl)sulfonamid; Arylsulfonamid; -NHNH₂; und -NHOH;
mit der Maßgabe, dass die Verbindungen der Formel I oder deren pharmazeutisch annehmbares Salz nicht die folgende Formel 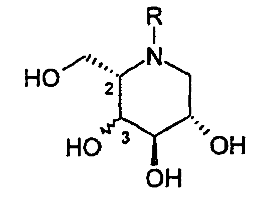 aufweisen, in der R für gerades oder verzweigtkettiges C₁₋₁₆-Alkyl, das gegebenenfalls mit C₃₋₇-Cycloalkyl substituiert ist und gegebenenfalls durch -O- unterbrochen ist, wobei der Sauerstoff von dem Ring-Stickstoff durch mindestens zwei Kohlenstoffatome getrennt ist, oder C₁₋₁₀-Alkylaryl steht, worin Aryl für Phenyl, Pyridyl, Thienyl oder Furyl steht, wobei Phenyl gegebenenfalls mit einem oder mehreren Substituenten substituiert ist, die ausgewählt sind aus F, Cl, Br, CF₃, OCF₃, OR¹ und geradem oder verzweigtkettigem C₁₋₆₋Alkyl; und
R¹ Wasserstoff oder gerades oder verzweigtkettiges C₁₋₆-Alkyl ist;
wobei die Zusammensetzung in einer Menge und über eine Zeit verabreicht wird, die ausreichen, um den männlichen Säuger unfruchtbar zu machen; und
(b) Aufhören mit der Verabreichung der Zusammensetzung an den Säuger, wodurch der Säuger fruchtbar gemacht wird; und
(c) ein- oder mehrmaliges Wiederholen der Schritte (a) und (b).

12. Verfahren nach Anspruch 11, in dem mindestens eines von R¹¹, R^{11'}, R¹², R^{12'}, R¹³, R^{13'} , R¹⁴, R^{14'}, R¹⁵ und R^{15'} für -CH₂OH steht.

13. Verfahren nach Anspruch 11 oder 12, in dem mindestens eines von R¹¹, R^{11'}, R¹², R^{12'}, R¹³, R^{13'}, R¹⁴, R^{14'}, R¹⁵ und R^{15'} für -OH steht.

14. Verfahren nach den Ansprüchen 11 bis 13, in dem R² eine lineare oder verzweigte C₁₋₁₈-Alkylgruppe ist.

15. Verfahren nach Anspruch 14, in dem R² eine lineare oder verzweigte C₄₋₉₋Alkylgruppe ist.

16. Verfahren nach den Ansprüchen 11 bis 15, in dem die Verbindung eine ist, die ausgewählt ist aus der Gruppe bestehend aus: 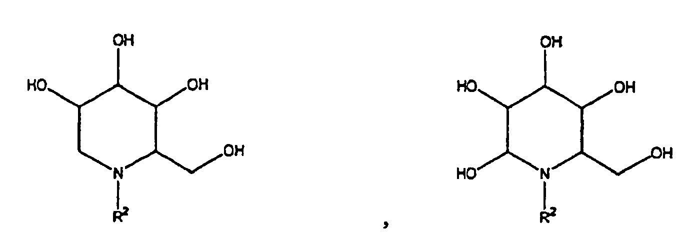 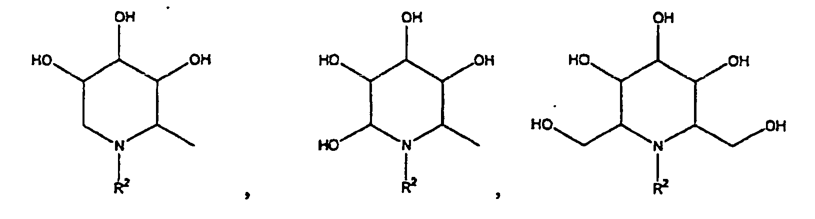 und deren Stereoisomeren.

17. Verfahren nach Anspruch 16, in dem die Verbindung eine ist, die ausgewählt ist aus der Gruppe bestehend aus Verbindungen, die in der folgenden Tabelle aufgeführt sind:

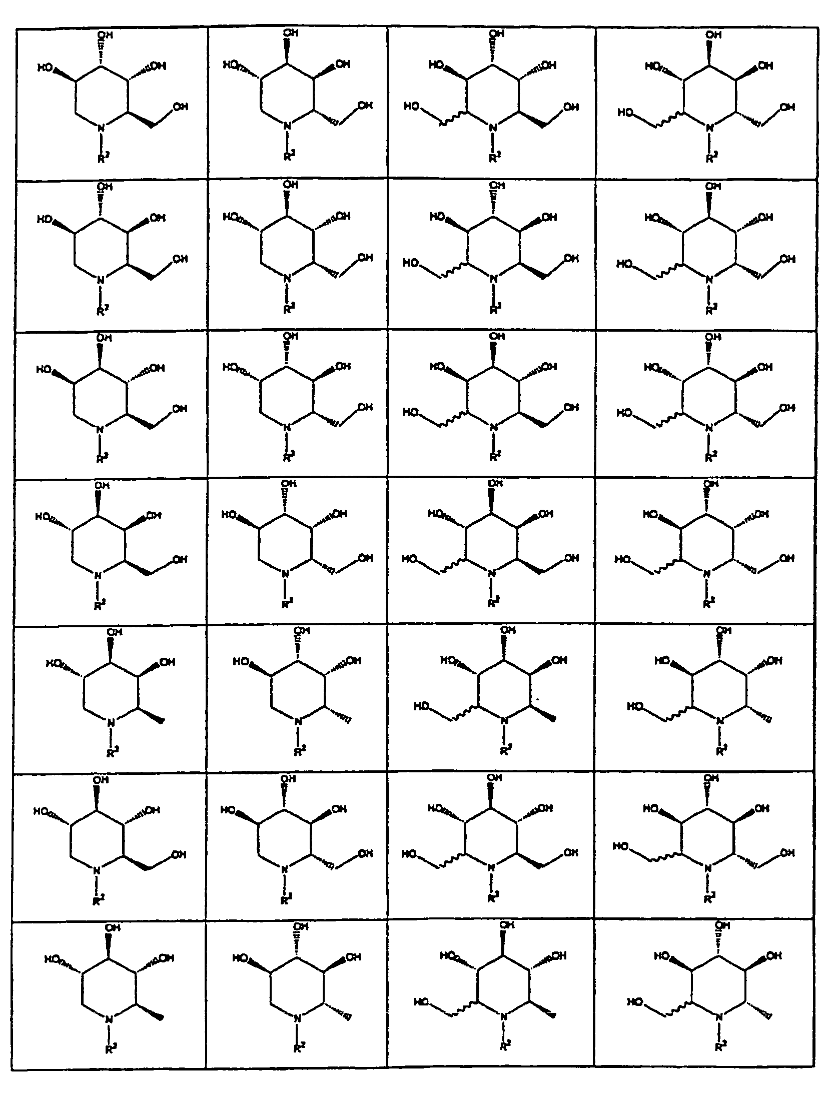

18. Verfahren nach Anspruch 17, in dem R² eine lineare oder verzweigte C₁₋₁₈₋Alkylgruppe ist, die gegebenenfalls mit C₁₋₆-Alkoxy substituiert ist.

19. Verfahren nach Anspruch 18, in dem die Verbindung eine ist, die ausgewählt ist aus der Gruppe bestehend aus: 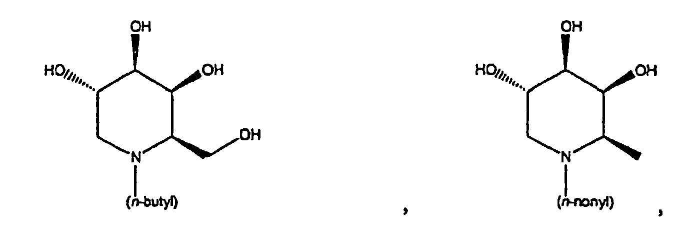 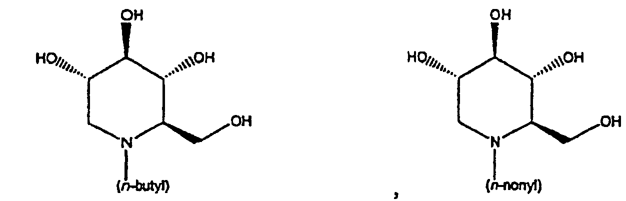 und

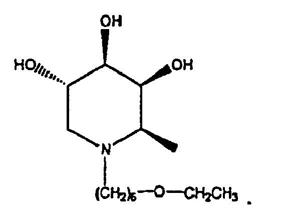

20. Verfahren nach den Ansprüchen 11 bis 19, weiter umfassend die aufeinanderfolgende Wiederholung der Schritte (a), (b) und (c).

21. Verfahren nach den Ansprüchen 1 bis 20, in dem der Säuger ein Mensch ist.

22. Verfahren nach den Ansprüchen 1 bis 20, in dem der Säuger ein Begleitart-Säuger ist.

23. Verfahren nach Anspruch 22, in dem der Säuger einer ist, der ausgewählt ist aus der Gruppe bestehend aus Hunden, Katzen und Pferden.

24. Verfahren nach den Ansprüchen 1 bis 20, in dem der Säuger ein Huftier ist.

25. Verfahren nach Anspruch 24, in dem der Säuger einer ist, der ausgewählt ist aus der Gruppe bestehend aus Vieh, Schafen, Ziegen, Wasserbüffel, Kamelen und Schweinen.

26. Verfahren nach den Ansprüchen 1 bis 20, in dem der Säuger einer ist, der aus schwanzlosen Affen, Affen, Lamas, Nagern und Kaninchen ausgewählt ist.

## Revendications

1. Procédé destiné à rendre réversiblement infécond un mammifère male fécond, comprenant les étapes consistant à :
(a) administrer audit mammifère une composition pharmaceutique comprenant un ou plusieurs excipients pharmaceutiquement acceptables et une quantité thérapeutiquement efficace d'un composé de formule I, ou d'un stéréoisomère, d'un sel pharmaceutiquement acceptable, ou d'un solvate de celui-ci : 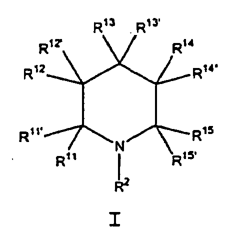 dans lequel chacun de R¹¹, R^{11'}, R¹², R^{12'}, R¹³, R^{13'}, R¹⁴, R^{14'}, R¹⁵ et R^{15'} est choisi, indépendamment des autres, dans le groupe constitué de -H, -OH, -F, -Cl, -Br, -I, -NH₂, un alkyle et dialkylamino, alkyle en C₁ à C₆, alcényle et alcynyle en C₂ à C₆ linéaire ou ramifié, aralkyle, alcoxy en C₁ à C₆ linéaire ou ramifié, aryloxy, aralcoxy, -(alkylène)oxy(alkyle), -CN, -NO₂, -COOH, -COO (alkyle), -COO(aryle), -C(O)NH(alkyle en C₁ à C₆), -C(O)NH(aryle), sulfonyle, (alkyle en C₁ à C₆)sulfonyle, arylsulfonyle, sulfamoyle, (alkyle en C₁ à C₆) sulfamoyle, (alkyle en C₁ à C₆) thio, (alkyle en C₁ à C₆) sulfonamide, arylsulfonamide, -NHNH₂, -NHOH, aryle et hétéroaryle ;
dans lequel chaque fragment alkyle, alcényle, alcynyle, aryle et hétéroaryle peut être éventuellement substitué avec un ou plusieurs groupes choisis indépendamment dans le groupe constitué de -OH, -F, -Cl, -Br, -I, -NH₂, alkyl- et dialkylamino, alkyle en C₁ à C₆, alcényle et alcynyle en C₂ à C₆ linéaire ou ramifié, aralkyle, alcoxy en C₁ à C₆ linéaire ou ramifié, aryloxy, aralcoxy, -(alkylène)oxy(alkyle), -CN, -NO₂, -COOH, -COO(alkyle), -COO(aryle), -C(O)NH(alkyle en C₁ à C₆), -C(O)NH(aryle), sulfonyle, (alkyle en C₁ à C₆)sulfonyle, arylsulfonyle, sulfamoyle, (alkyle en C₁ à C₆) sulfamoyle ; (alkyle en C₁ à C₆) thio, (alkyle en C₁ à C₆) sulfonamide, arylsulfonamide, -NHNH₂ et -NHOH ;
et dans lequel au moins deux de R¹¹, R^{11'}, R¹², R^{12'}, R¹³, R^{13'}, R¹⁴, R^{14'}, R¹⁵ et R^{15'} sont choisis dans le groupe constitué de -CH₃, -CH₂OH et -OH,
R² et un substituant choisi dans le groupe constitué d'un alkyle en C₁ à C₁₈, alcényle et alcynyle en C₂ à C₁₈ linéaire ou ramifié, et aralkyle ;
dans lequel chaque fragment alkyle, alcényle, alcynyle et aryle peut être éventuellement substitué par un ou plusieurs groupes choisis indépendamment dans le groupe constitué de -OH, -F, -Cl, -Br, -I, -NH₂, un alkyl- et dialkylamino, alkyle en C₁ à C₆, alcényle et alcynyle en C₂ à C₆ linéaire ou ramifié, aralkyle, alcoxy en C₁ à C₆ linéaire ou ramifié, aryloxy, aralcoxy, -CN, -NO₂, -COOH, -COO(alkyle), -COO(aryle), -C(O)NH(alkyle en C₁ à C₆), -C(O)NH(aryle), sulfonyle, (alkyle en C₁ à C₆) sulfonyle, arylsulfonyle, sulfamoyle, (alkyle en C₁ à C₆) sulfamoyle ; (alkyle en C₁ à C₆) thio, (alkyle en C₁ à C₆) sulfonamide, arylsulfonamide, -NHNH₂ et -NHOH ;
à condition que les composés de formule I ou leurs sels pharmaceutiquement acceptables n'aient pas la formule suivante 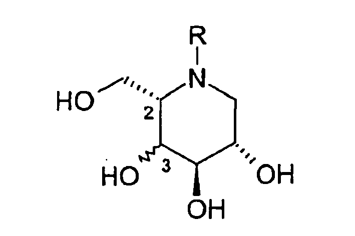 dans laquelle R est un alkyle en C₁ à C₁₆ linéaire ou ramifié, éventuellement substitué par un cycloalkyle en C₃ à C₇, et éventuellement interrompu par -O-, l'oxygène étant séparé de l'azote du cycle par au moins deux atomes de carbone, ou alkylaryle en C₁ à C₁₀
dans lequel aryle est phényle, pyridyle, thiényle ou furyle, dans lequel phényle est éventuellement substitué par un ou plusieurs substituants choisis parmi F, Cl, Br, CF₃, OCF₃, OR¹ et alkyle en C₁ à C₆ à chaîne linéaire ou ramifiée ; et
R¹ est un hydrogène ou alkyle en C₁ à C₆ à chaîne linéaire ou ramifiée ;
ladite composition étant administrée en une quantité et pendant un temps suffisants pour rendre ledit mammifère male infécond ; et
(b) cesser d'administrer ladite composition audit mammifère, pour rendre ledit mammifère fécond.

2. Procédé selon la revendication 1, dans lequel au moins un de R¹¹, R^{11'}, R¹², R^{12'}, R¹³, R^{13'}, R¹⁴, R^{14'}, R¹⁵ et R^{15'} est -CH₂OH.

3. Procédé selon la revendication 1 ou 2, dans lequel au moins un de R¹¹, R^{11'}, R¹², R^{12'}, R¹³, R^{13'}, R¹⁴, R^{14'}, R¹⁵ et R^{15'} est -OH.

4. Procédé selon les revendications 1 à 3; dans lequel R² est un groupe alkyle en C₁ à C₁₈ linéaire ou ramifié.

5. Procédé selon la revendication 4, dans lequel R² est un groupe alkyle en C₄ à C₉ linéaire ou ramifié.

6. Procédé selon les revendications 1 à 5, dans lequel le composé est choisi dans le groupe constitué de : 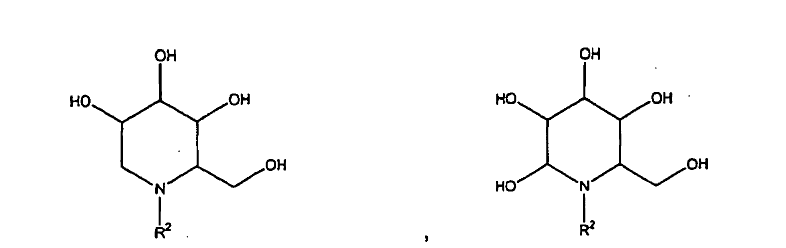 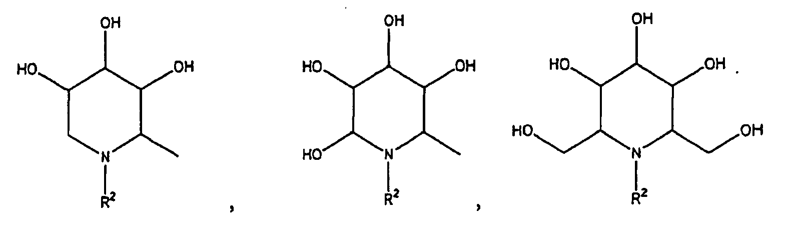 et des stéréoisomères de ceux-ci.

7. Procédé selon la revendication 6, dans lequel le composé est choisi dans le groupe constitué des composés présentés dans le tableau suivant :

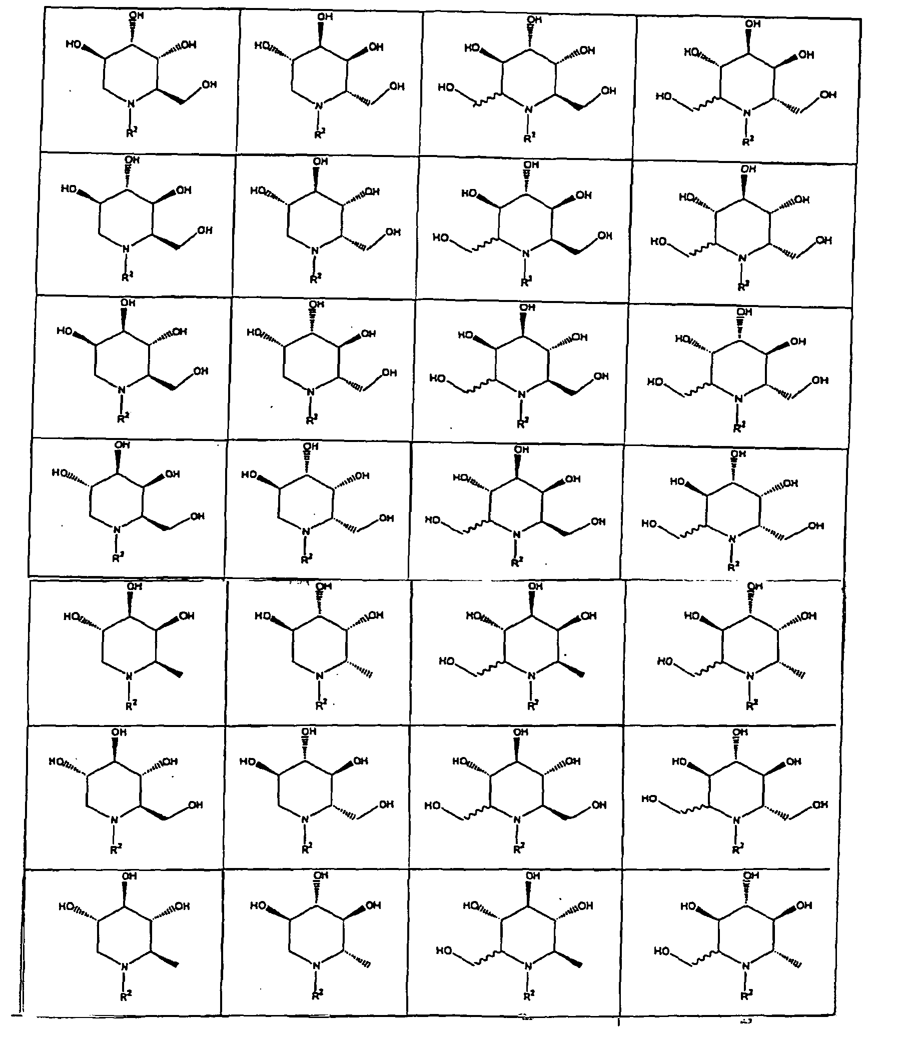

8. Procédé selon la revendication 7, dans lequel R³ est un groupe alkyle en C₁ à C₁₈ linéaire ou ramifié éventuellement substitué avec un alcoxy en C₁ à C₆.

9. Procédé selon la revendication 8, dans lequel le composé est choisi dans le groupe constitué de : 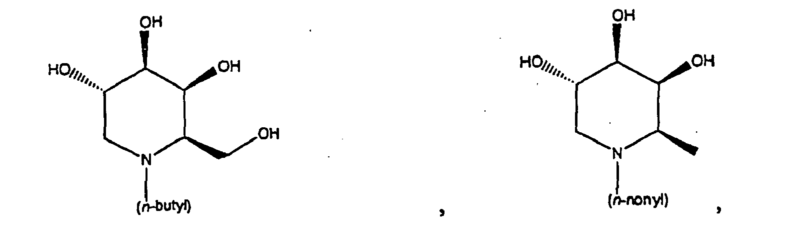 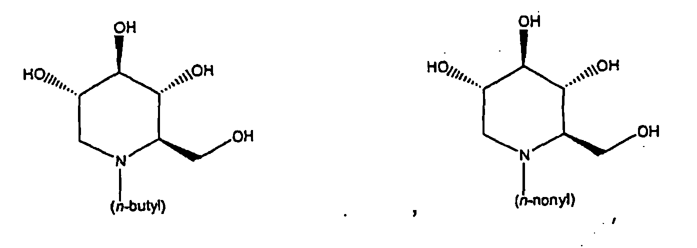 et

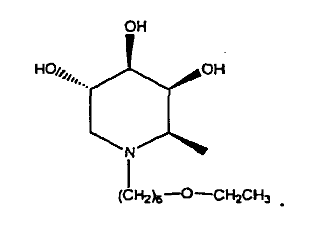

10. Procédé selon les revendications 1 à 9, comprenant en outre les étapes consistant à répéter séquentiellement les étapes (a) et (b).

11. Procédé destiné à rendre réversiblement infécond un mammifère male fécond, comprenant les étapes consistant à :
(a) administrer audit mammifère une composition pharmaceutique comprenant un ou plusieurs excipients pharmaceutiquement acceptables et une quantité thérapeutiquement efficace d'un composé de formule I, ou d'un stéréoisomère, d'un sel pharmaceutiquement acceptable, ou d'un solvate de celui-ci : 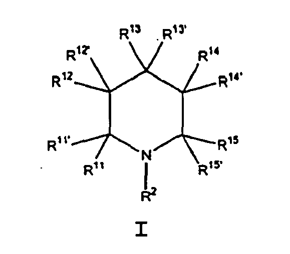 dans lequel chacun de R¹¹, R^{11'}, R¹², R^{12'}, R¹³, R^{13'}, R¹⁴, R^{14'}, R¹⁵ et R^{15'} est choisi, indépendamment des autres, dans le groupe constitué de -H, -OH, -F, -Cl, -Br, -I, -NH₂, un alkyle et dialkylamino, alkyle en C₁ à C₆, alcényle et alcynyle en C₂ à C₆ linéaire ou ramifié, aralkyle, alcoxy en C₁ à C₆ linéaire ou ramifié, aryloxy, aralcoxy, -(alkylène)oxy(alkyle), -CN, -NO₂, -COOH, -COO(alkyle), -COO(aryle), -C(O)NH(alkyle en C₁ à C₆), -C(O)NH(aryle), sulfonyle, (alkyle en C₁ à C₆) sulfonyle, arylsulfonyle, sulfamoyle, (alkyle en C₁ à C₆) sulfamoyle, (alkyle en C₁ à C₆) thio, (alkyle en C₁ à C₆) sulfonamide, arylsulfonamide, -NHNH₂, -NHOH, aryle et hétéroaryle ;
dans lequel chaque fragment alkyle, alcényle, alcynyle, aryle et hétéroaryle peut être éventuellement substitué avec un ou plusieurs groupes choisis indépendamment dans le groupe constitué de -OH, -F, -Cl, -Br, -I, -NH₂, alkyl- et dialkylamino, alkyle en C₁ à C₆, alcényle et alcynyle en C₂ à C₆ linéaire ou ramifié, aralkyle, alcoxy en C₁ à C₆ linéaire ou ramifié, aryloxy, aralcoxy, -(alkylène)oxy(alkyle), -CN, -NO₂, -COOH, -COO(alkyle), -COO(aryle), -C(O)NH (alkyle en C₁ à C₆), -C(O)NH(aryle), sulfonyle, (alkyle en C₁ à C₆) sulfonyle, arylsulfonyle, sulfamoyle, (alkyle en C₁ à C₆) sulfamoyle ; (alkyle en C₁ à C₆) thio, (alkyle en C₁ à C₆) sulfonamide, arylsulfonamide, -NHNH₂ et -NHOH ;
et dans lequel au moins deux de R¹¹, R^{11'}, R¹², R^{12'}, R¹³, R^{13'}, R¹⁴, R^{14'}, R¹⁵ et R^{15'} sont choisis dans le groupe constitué de -CH₃, -CH₂OH et -OH,
R² et un substituant choisi dans le groupe constitué d'un alkyle en C₁ à C₁₈, alcényle et alcynyle en C₂ à C₁₈ linéaire ou ramifié, et aralkyle ;
dans lequel chaque fragment alkyle, alcényle, alcynyle et aryle peut être éventuellement substitué par un ou plusieurs groupes choisis indépendamment dans le groupe constitué de -OH, -F, -Cl, -Br, -I, -NH₂, un alkyl- et dialkylamino, alkyle en C₁ à C₆, alcényle et alcynyle en C₂ à C₆ linéaire ou ramifié, aralkyle, alcoxy en C₁ à C₆ linéaire ou ramifié, aryloxy, aralcoxy, -CN, -NO₂ -COOH, -COO(alkyle), -COO(aryle), -C(O)NH(alkyle en C₁ à C₆), -C(O)NH(aryle), sulfonyle, (alkyle en C₁ à C₆) sulfonyle, arylsulfonyle, sulfamoyle, (alkyle en C₁ à C₆) sulfamoyle ; (alkyle en C₁ à C₆) thio, (alkyle en C₁ à C₆) sulfonamide, arylsulfonamide, -NHNH₂ et -NHOH ;
à condition que les composés de formule I ou leurs sels pharmaceutiquement acceptables n'aient pas la formule suivante 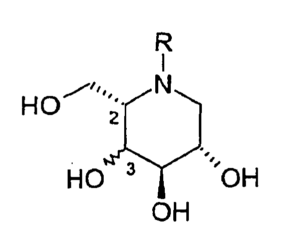 dans laquelle R est un alkyle en C₁ à C₁₆ linéaire ou ramifié, éventuellement substitué par un cycloalkyle en C₃ à C₇, et éventuellement interrompu par -O-, l'oxygène étant séparé de l'azote du cycle par au moins deux atomes de carbone, ou alkylaryle en C₁ à C₁₀ dans lequel aryle est phényle, pyridyle, thiényle ou furyle, dans lequel phényle est éventuellement substitué par un ou plusieurs substituants choisis parmi F, Cl, Br, CF₃, OCF₃, OR¹ et alkyle en C₁ à C₆ à chaîne linéaire ou ramifiée ; et
R¹ est un hydrogène ou alkyle en C₁ à C₆ à chaîne linéaire ou ramifiée ;
ladite composition étant administrée en une quantité et pendant un temps suffisants pour rendre ledit mammifère male infécond ; et
(b) cesser d'administrer ladite composition audit mammifère, pour rendre ledit mammifère fécond ; et
(c) répéter les étapes (a) et (b) une ou plusieurs fois.

12. Procédé selon la revendication 11, dans lequel au moins un de R¹¹, R^{11'}, R¹², R^{12'}, R¹³, R^{13'}, R¹⁴, R^{14'}, R¹⁵ et R^{15'} est -CH₂OH.

13. Procédé selon la revendication 11 ou 12, dans lequel au moins un de R¹¹, R^{11'}, R¹², R^{12'}, R¹³, R^{13'}, R¹⁴, R^{14'}, R¹⁵ et R^{15'} est -OH.

14. Procédé selon les revendications 11 à 13, dans lequel R² est un groupe alkyle en C₁ à C₁₈ linéaire ou ramifié.

15. Procédé selon la revendication 14, dans lequel R² est un groupe alkyle en C₄ à C₉ linéaire ou ramifié.

16. Procédé selon les revendications 11 à 15, dans lequel le composé est choisi dans le groupe constitué de : 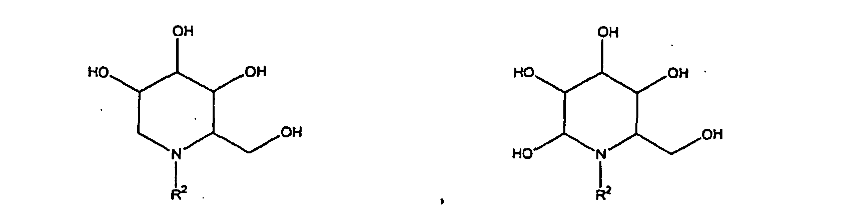 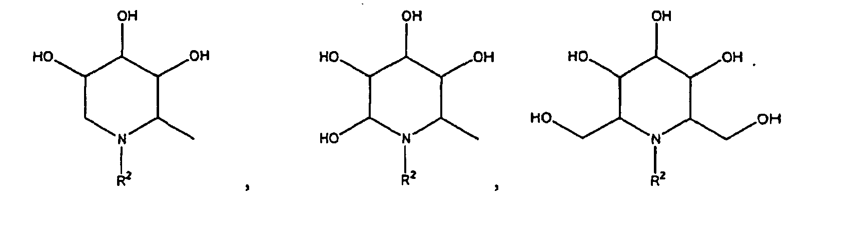 et des stéréoisomères de ceux-ci.

17. Procédé selon la revendication 16, dans lequel le composé est choisi dans le groupe constitué des composés présentés dans le tableau suivant :

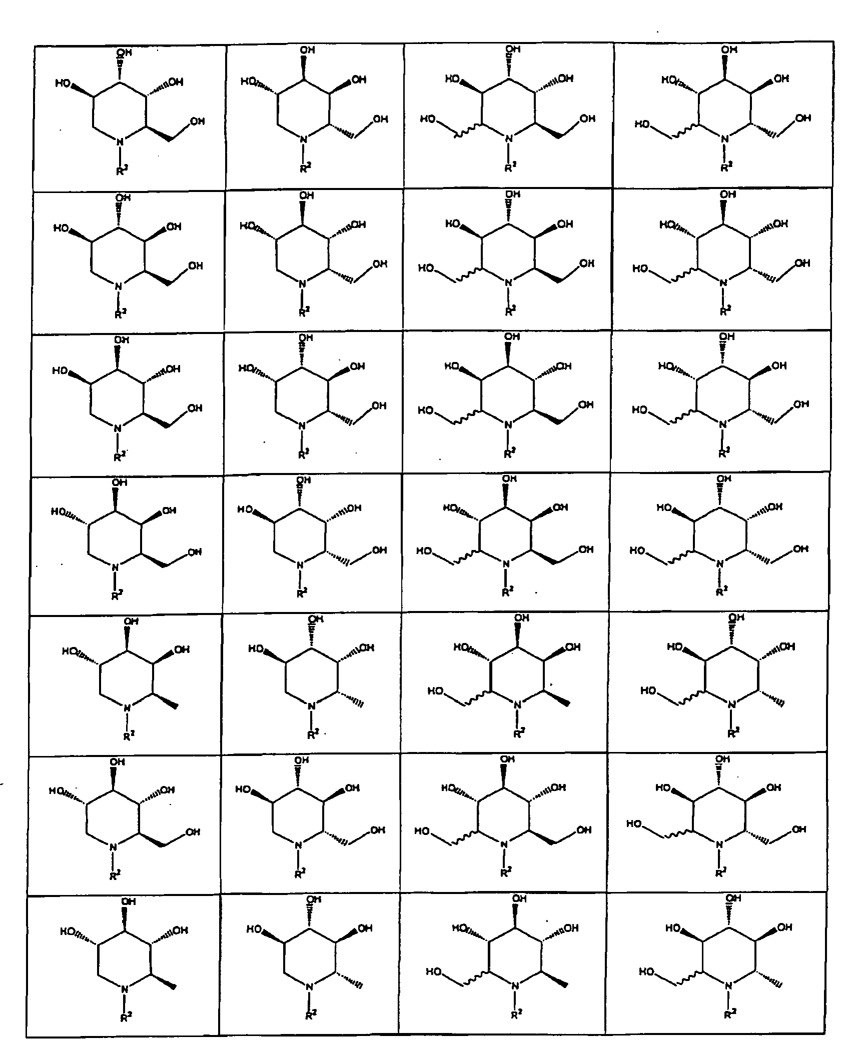

18. Procédé selon la revendication 17, dans lequel R² est un groupe alkyle en C₁ à C₁₈ linéaire ou ramifié éventuellement substitué avec un alcoxy en C₁ à C₆.

19. Procédé selon la revendication 18, dans lequel le composé est choisi dans le groupe constitué de : 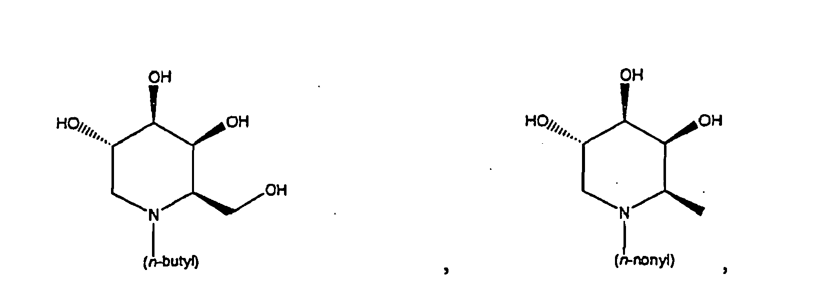 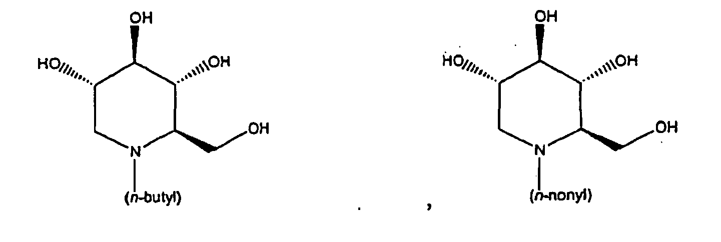 et

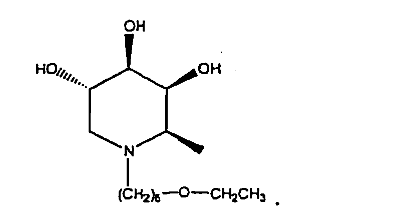

20. Procédé selon les revendications 11 à 19, comprenant en outre les étapes consistant à répéter séquentiellement les étapes (a), (b) et (c).

21. Procédé selon les revendications 1 à 20 dans lequel ledit mammifère est un être humain.

22. Procédé selon les revendications 1 à 20 dans lequel ledit mammifère est un mammifère de compagnie.

23. Procédé selon la revendication 22, dans lequel ledit mammifère est choisi dans le groupe constitué des chiens, des chats et des chevaux.

24. Procédé selon les revendications 1 à 20, dans lequel ledit mammifère est un ongulé.

25. Procédé selon la revendication 24, dans lequel ledit mammifère est choisi dans le groupe constitué du bétail, des moutons, des chèvres, des buffles des Indes, des chameaux et des porcs.

26. Procédé selon les revendications 1 à 20, dans lequel ledit mammifère est choisi parmi les singes, les lamas, les rongeurs et les lapins.
